# EUROPÄISCHE PATENTSCHRIFT

(11) **EP 0 836 522 B1**
(45) Veröffentlichungstag und Bekanntmachung des Hinweises auf die Patenterteilung: **07.04.2004**
(21) Anmeldenummer: 96922905.3
(22) Anmeldetag: 03.07.1996
(51) Int. Cl.: A63J 5/00, A61L 9/12

(54) **VORRICHTUNG UND VERFAHREN ZUR BEREITSTELLUNG VON IN DIE LUFT ABZUGEBENDEN STOFFEN**
DEVICE AND PROCESS FOR DELIVERING SUBSTANCES FOR DISPERSAL IN THE AIR
PROCEDE ET DISPOSITIF POUR ACHEMINER DES SUBSTANCES A DIFFUSER DANS L'AIR

(30) Priorität: 03.07.1995 DE 19524193; 17.07.1995 DE 19526002; 16.08.1995 DE 19530111; 08.12.1995 DE 19545950; 06.03.1996 DE 19608708; 02.07.1996 DE 19626602
(43) Veröffentlichungstag der Anmeldung: 22.04.1998
(73) Patentinhaber: WITTEK, Götz-Ulrich, London W1R 5FA (GB)
(72) Erfinder: WITTEK, Götz-Ulrich, London W1R 5FA (GB)
(74) Vertreter: Diehl, Hermann, Dr. Dipl.-Phys.
(86) Internationale Anmeldenummer: PCT/EP1996/002925
(87) Internationale Veröffentlichungsnummer: WO 1997/002076

(56) Entgegenhaltungen:
- WO-A-93/08676
- US-A- 4 603 030
- US-A- 5 273 690

## Beschreibung

Die vorliegende Erfindung betrifft eine Vorrichtung und ein Verfahren zur Bereitstellung von in die Luft oder in Luftgemische abzugebenden Stoffen.
Diese abzugebenden Stoffe sind insbesondere unterschiedliche Düfte. Eine Duftdarbietung erfolgt vorzugsweise zu visuellen und/oder akustischen Reizen und anderen Ereignissen. Neben Düften können dies bei einer sonstigen Darbietung auch Mikromengen von in Aerosolform benötigten, anderen Stoffen sein. Die Substanzen können ebenfalls in gasförmiger Form vorliegen. Wenn im folgenden von "Luft" die Rede ist, können damit auch Luftgemische gemeint sein.

Die Erfindung befaßt sich insbesondere auch mit einem Verfahren und einer Vorrichtung zur Erhöhung der sinnlichen Wahrnehmung von visuellen und/oder akustischen Darbietungen, insbesondere für dezentrale Medien, wie z.B. bei der Aufführung von Fernsehfilmen, Videofilmen, Radiosendungen oder Musikdarbietungen und dergleichen, wobei ben Zuschauern bzw. Zuhörern synchron zur Darbietung von bestimmten, visuellen, und/oder akustischen Ereignissen (z.B. Filmszenen) dazu passende Düfte zugeführt werden.

Ein derartiges Verfahren und zugehörige Vorrichtungen sind in der Patentanmeldung PCT/EP92/02446 des Anmelders beschrieben, hier insbesondere in der Beschreibung der Figuren 16 und 17.

Aus der US-A-5273690 ist eine Lufterfrischervorrichtung bekannt, welche beispielsweise zusammen mit einer Klimaanlage zur Luftverbesserung in Räumen eingesetzt werden kann. Die Lufterfrischervorrichtung weist einen Träger mit einer Vielzahl von Düften auf, welche in Reihen oder Säulen in voneinander abgetrennten Zellen oder Kompartimenten angeordnet sind. Der Träger umfaßt für jede Zelle oder jedes Kompartiment aufbrechbare Wände, welche so ausgestaltet sind, daß freigesetzte Düfte in einem ankommenden Luftstrom abgegeben werden können. Der Träger kann mit Befestigungselementen an einer Stützstruktur befestigt werden, so daß freigesetzte Düfte mittels eines Luftstromes in einem gesamten Raum verteilt werden können.

Der vorliegenden Erfindung liegt die Aufgabe zugrunde, eine Vorrichtung und ein Verfahren zur Bereitstellung von in die Luft abzugebenden Stoffen anzugeben, wobei die Vorrichtung kleine und handliche Abmessungen aufweist und den abzugebenden Stoff in leicht aktivierbarer Form enthält. Diese Aufgabe löst die Erfindung durch die in den unabhängigen Patentansprüchen 1, 9 und 30 angegebenen Vorrichtungen. Bevorzugte Ausgestaltungen und Aspekte der Erfindung ergeben sich aus den abhängigen Patentansprüchen, sowie aus der Beschreibung und den Zeichnungen.

Erfindungsgemäß angegeben wird eine Vorrichtung zur Bereitstellung von in die Luft abzugebenden Stoffen, insbesondere von Duftstoffen mit einem flachen, scheiben- oder plattenförmigen Grundkörper, der im wesentlichen parallel zu seiner Ober- und/oder Unterseite von mehreren separaten Kanälen durchsetzt ist, die zur Aufnahme der in die Luft abzugebenden Stoffe dienen und je eine Ein- und Auslaßöffnung enthalten und von einem der Einlaßöffnung zugeführten Gasstrom durchströmbar sind, wobei die Ein- und Auslaßöffnung zumindest eines Kanals bis zur Abgabe des Stoffes gasdicht verschlossen und/oder der Stoff in zumindest einen Kanal in einem Speicher gasdicht eingebracht ist, der erst zum Abgabezeitpunkt des Stoffes diesen freisetzt.

Weiterhin betrifft die Erfindung eine Vorrichtung zur Bereitstellung von in die Luft abzugebenden Stoffen, insbesondere Duftstoffen,
gekennzeichnet durch
- zumindest einen mit wenigstens einer Luftaustauschöffnung versehenen Hohlkörper, dessen Inneres von einer Gasströmung durchspülbar ist;
- zumindest einen in dem Hohlkörper angeordneten Speicher, für den in die Umgebungsluft abzugebenden Stoff, der von einem Trägermaterial so eingeschlossen ist, daß der Stoff durch Zerstörung des Trägermaterials freisetzbar ist, wobei hierfür Mittel vorgesehen sind, die entweder in der Vorrichtung selbst angebracht sind oder getrennt von der Vorrichtung derart vorgesehen sind, daß sie bei Aktivierung der Abgabe mit der Vorrichtung in Wirkverbindung bringbar sind, wobei die Mittel vorzugsweise so ausgebildet sind, daß sie beim Einlegen der Vorrichtung in ein Gerät zur Abgabe der gasförmigen Stoffe wirksam werden.

Unter Zerstörung des Trägermaterials ist zu verstehen, daß die Integrität des Trägermaterials derart verändert wird, daß die abzugebenden Stoffe freiliegen. Dies kann durch Auseinanderreißen eines Bestandteils eines Laminats, Zerbrechen und andersartige Unterteilungen erfolgen.

Gemäß eines weiteren Aspekts betrifft die vorliegende Erfindung einen Speicher mit Duftkapseln, der sich bei einem Einströmen von Luft durch das selbständige Ablösen einer Trennfolie von selbst aktiviert.

Ein weiterer Aspekt der Erfindung betrifft einen Mikro-Duftspeicher, der in dezentral angeordneten Duftgebern abspielbar ist, wobei der Mikro-Duftspeicher diskettenförmig ausgestaltet sein kann.

Die erfindungsgemäße Vorrichtung zur Bereitstellung von in die Luft abzugebenden Stoffen weist die Vorteile auf, sehr klein, handlich und leicht verpackbar und verschickbar zu sein. Weitere Vorteile sind, daß die Vorrichtung mehrere Stoffe aufnehmen kann, die nicht ausduften und sich nicht vermischen können. Die freigegebenen Stoffe sind darüberhinaus leicht aktivierbar und beim Transport gut geschützt.

Die vorliegende Erfindung stellt unter anderen Verfahren bzw. eine Vorrichtung zur Verfügung, bei denen die verschiedenen, zu einem einzelnen Film zugehörigen Duftvorräte (Duftsets) in einer Weise hergestellt und aufbewahrt werden, welche bei niedrigen Herstellkosten sowohl eine Lagerung dieser Düfte (vorzugsweise 20-50 verschiedene Düfte pro Film) auf extrem kleinem Raum, (Kompakt-Diskette), als auch deren perfekte Konservierung und schnelle, störungsfreie Transportierbarkeit gewährleistet.

Ein weiterer Aspekt der Erfindung ist, daß ein Hartmaterialspeicher für in die Luft abgebbare Stoffe, insbesondere Duftstoffe zur Verfügung gestellt wird, der aus leicht trennbaren Hälften besteht.

Gemäß eines weiteren Aspekts der Erfindung wird ein flacher Hartmaterialschutz angegeben, der gespeicherte, in die Luft abgebbare Stoffe, z.B. Duftstoffe, vor äußeren Umwelteinflüssen, wie Druck, Stoß, Wärme und Beschädigung schützt.

Ein weiterer Aspekt ist ein flacher Duftstoffspeicher mit versiegelten, abspielbaren Duftstoffen, wobei die Versiegelungen erst unmittelbar vor dem Abspielen entfernt werden.

Allgemein gesprochen stellt die Erfindung ein Flachkonservierungssystem für Multiduftsets dar, insbesondere einen flachen oder scheibenförmigen Hartmaterialschutz für druckempfindliche und/oder licht- oder wärmeempfindliche Duftstoffsets.

Ein weiterer Aspekt ist ein flacher Hartmaterial-Versandspeicher mit darin angeordneten Schutzröhren zum Schutz druckempfindlicher Duftstoffe.

Ein weiterer Aspekt der vorliegenden Erfindung ist, daß ein scheibenartiger Duftstoffspeicher angegeben wird, der für den druck- temperatur-, feuchtigkeits- und lichtgeschützten Transport von verkapselten Duftstoffen geeignet ist.

Außerdem wird ein Verfahren zur Freisetzung von in die Luft abzugebenden Stoffen, insbesondere Duftstoffen angegeben, wobei die Stoffe mikroverkapselt in ein Trägermaterial eingebettet sind, welches dadurch gekennzeichnet ist, daß die in dem Trägermaterial eingebetteten Duftstoffmikrokapseln durch die Bewegungsenergie eines Gases aufgebrochen werden kann.

Die Erfindung schafft somit ein Mikro-Duftspeichersystem (Duftdisketten) für die automatische Abspielung ereignisbezogener Duftsets in dezentral verteilten Duftgebern (Duft-Playern), wodurch eine dezentrale und zugleich zeitsynchrone Duftbegleitung von Filmen, Musik und anderen Ereignissen, ermöglicht wird.

Gemäß einer bevorzugten Ausgestaltung des erfindungsgemäßen Verfahrens zur minimalisierten Speicherung und dezentralen, synchronen Darbietung von Düften zu visuellen und/oder akustischen Reizen werden die mit den benötigten Duftstoffen ausgestatteten Mikro-Duftspeicher in der Form sehr flacher Kompaktdisketten 1 hergestellt, wie dies etwa von der Musik-CD her bekannt ist.

Diese sehr kleine und flache Ausführungsform der Duft-Träger hat hierbei den grundsätzlichen Vorteil, daß sie trotz relativ großer Duftkontaktflächen, nur wenig Raum einnimmt und dadurch eine schnelle und massenweise Verteilung der jeweils zu einem bestimmten Film zugehörigen Duftsets an ein großes Publikum ermöglicht.
So ist es bei Duftset-Trägern dieser Art und Form möglich, diese z.B. einer Tages- oder Fernsehzeitung oder einem Produkt, z.B. einer Packung Kaffee, etc. beizukleben. Auf diese Weise können sämtliche, zu einem Fernsehfilm zugehörigen Düfte (Duftsets) zum Zeitpunkt der Ausstrahlung allen interessierten Zuschauern vorliegen, ohne, daß sich jeder einzelne Fernsehzuschauer für einen Film, den er als Duftversion sehen möchte, jeweils eine Duft-CD kaufen muß.
Über die schnelle, dezentrale Verteilung genau zu einem Film komponierter und perfekt geschützter Duftsets, die als flache Duft-Kompaktdiskette einer Tages-, bzw. Fernsehzeitung oder einer Postsendung beiliegen, wird es hierdurch machbar, daß die zu einem Fernsehfilm oder sonstigen Medienereignis zugehörigen Düfte (Duftsets) zum Zeitpunkt der Ausstrahlung bereits allen interessierten Zuschauern perfekt konserviert vorliegen, ohne daß jeder einzelne Fernsehzuschauer in ein Geschäft gehen muß, um sich für einen Film, den er als Duftversion sehen möchte, ein neues, entsprechendes Duftset zu kaufen.

Hierdurch wird im Bereich der Medien erstmals die schnelle Verteilung, langfristige Bevorratung und schließlich die dezentrale, zeitsynchrone Einspielung vieler verschiedener, jeweils genau zu einem Film passender Duftsets für ein dezentral verteiltes Massenpublikum ermöglicht.

Hierbei wird erstmalig auch eine Technik realisiert, bei welcher der Trägerstoff (z.B. Luft) den verschlossenen und konservierten Duftstoff selbsttätig aus dem verschlossenen Zustand heraus öffnet, nach und nach herauslöst und mit sich führt.
Die Duftstoffe verbleiben bei dieser Technik also bis zum unmittelbaren Zeitpunkt der Vorführung unter Luftabschluß, so daß trotz Unterbringung auf kleinstem Raum vorzeitige Alterungsprozesse zuverlässig vermieden werden.

Eine weitere Anwendung dieser Disketten kann auch in der Medizintechnik für die Applikation von sehr kleinen, kontrollierten Medikamentenmengen liegen.

Im Unterschied zu den flachen und binären Informationsträgern Computer-Diskette und Musik-CD, ist eine Lagerung und Verteilung von Düften jedoch an die stoffliche Form der Düfte gebunden.
Vorzugsweise werden hierbei die unterschiedlichen Vorräte von Mikrodüften auf der Innenseite einer der beiden Hälften einer derartigen Diskette in Form einer bestimmten Art von Duftspuren aufgetragen (Duftbahn-Anordnung).

Derartige Duftbahn-Anordnungen können auch in mehreren Schichten übereinander in einer derartigen Diskette gelagert sein, da die einzelnen Schichten extrem dünn sind und dadurch nur sehr wenig Raum in Anspruch nehmen. Die einzelnen Spuren einer derartigen Duftbahn-Anordnung werden hierbei durch bestimmte Muster von Trennschnitten gegeneinander abgeteilt. Diese Trennschnitte können dabei als Muster in die jeweilige Disketten-Hälfte mit eingepresst werden, oder als Vertiefungen und Erhöhungen auf dem Gegenstück der anderen Disketten-Hälfte, wobei sich die einzelnen, duftgebenden Spuren schließlich durch den Herstellungsprozeß ergeben, während die Disketten-Hälften auf eine bestimmte Art und Weise zusammengefügt werden.

Um während des Aufbewahrungszustandes der Diskette eine Durchmischung der einzelnen Düfte und Aromen aus den Duft-Bahnen zu verhindern, werden diese Bahnen vorzugsweise so gestaltet, daß bei der Produktion der Diskette kleine, voneinander abgetrennte Schutzkanäle entstehen, welche die einzelnen Duftspuren voneinander abgrenzen.

Innerhalb der abgetrennten Duftkanäle sind die Duftstoffe vorzugsweise noch ein weiteres Mal in duftführenden Schlitzkanälen versiegelt, um neben der Einwirkung durch Fremddüfte ebenfalls vor der Einwirkung durch Oxydationsprozesse geschützt zu sein.

Eine dritte Versiegelung der Duftstoffe kann hierbei noch durch eine Verkapselung erreicht werden, indem die Duftstoffe innerhalb der duftführenden Schlitzkanäle ein weiteres mal in sehr kleinen Mikrokapseln verschlossen werden, welche gleichzeitig mit der Öffnung der Schlitzkanäle aufplatzen.

Sowohl der Durchmesser der Trennkanäle, als auch die Erhebung durch die Zweitversiegelung der Düfte ist hierbei vorzugsweise derart ausgestaltet, daß die Duft-CD insgesamt nur eine Dicke erreicht, die im Bereich von handelsüblichen Musik-CD's / CD-ROM's liegt, oder zumindest so wenig darüber, daß diese Duft-CD's noch problemlos einer Zeitung beigeklebt werden können.

Soweit nun ein Spielfilm über das TV oder ein Videogerät abgespielt wird, werden hierbei die Signale, welche einem Duft zugeordnet sind, durch den entsprechenden Fernsehsender oder über das Speichermedium, zusammen mit den Bild- und Tonsignalen des Spielfilms, abgespielt, wie dies bereits in der früheren PCT/EP92/02446 (Fig.16 u. 17) des Anmelders beschrieben ist.

Hierbei wirken diese Signale direkt oder indirekt auf ein Abspielgerät, den sogenannten Duft-Player ein, in welchen die oben beschriebenen Duftdisketten vor Beginn des Spielfilms gelegt werden.

Soweit über ein Duftsignal nun eine dieser Duftspuren aktiviert wird, wird ein Trägermedium, welches z.B. Luft-, oder ein bestimmtes Luftgemisch sein kann, durch eine kleine Pumpe angetrieben und über einen Mikro-Einfüllstutzen in den entsprechenden Duftkanal auf der Duftdiskette hineingepumpt. Durch den Druck, welches das Trägermedium mit sich bringt, werden innerhalb des Duftkanals nun bestimmte, sehr dünne, duftführende Schichten aktiviert.
Die Signale zur Ansteuerung der Düfte können auch weitere Merkmale einer bedufteten Filmvorführung beeinflussen, wie z.B. die Menge, der dabei durch einen Duftträger fließenden Luft, die Temperatur des abgegebenen Duft-Luft-Gemisches die Länge des Duftintervalls, eventuell beabsichtigte Vermischungen von Düften durch deren gleichzeitige Aktivierung oder eine Überblendung von einem in einen anderen Duft etc.

Sofern es beabsichtigt wird, in einer Abwandlung des vorgesehenen Equipments auch duftbegleitete Theater-, Musik-, oder Filmaufführungen in öffentlichen Theatern etc. aufzuführen, erleichtert es die Erfindung desweiteren, den Aufwand für den Umbau von derartigen Gebäuden (wie in der PCT/EP92/02446 beschrieben) durch Weglassen der dazu normalerweise erforderlichen Duftleitungs-Einbauten auf ein Mindestmaß zu reduzieren. Statt der zentralen Duftabspielvorrichtung und dem kompletten Leitungssystem wird hierbei an jedem gewünschten Platz ein kleines Abspielgerät befestigt. Dies wird insbesondere dann wichtig, wenn geplant ist, nur bestimmte Aufführungen über einen begrenzten Zeitraum hinweg mit Düften zu begleiten, welches die Kosten für einen kompletten Umbau des Theaters u.U. nicht rechtfertigen würde. Auch eine Duftbegleitung von open air Aufführungen kann diese Vorrichtung ermöglichen.

Mit der Schaffung eines neuartigen und durch weitestgehende Miniaturisierung leicht über Massenmedien, wie z.B. Zeitungen, verteilbaren und automatisch abspielbaren Multi-Duft-Speichersystems, ermöglicht es die Erfindung desweiteren, dieses System für jegliche, dezentrale Anwendung, die gewünscht wird, zu verwenden.

So ist es möglich, dieses System neben der Anwendung für Filme auch für andere Medien, wie die Werbung zu verwenden, oder etwa für Nahrungsmittel- und Getränkeautomaten, Computerspiele oder für einen duftbegleiteten Online-Einkauf, für Musik-CD's, Produktinformations-Einheiten (z.B. Informations-Computer am sog. "point of sale") sowie für jegliche Art von Vorführung, welche zentral oder dezentral durch die Hinzufügung von Düften gesteigert werden kann.

Wie die bereits gezeigten anderen, dezentralen Anwendungen, bieten auch die in jüngster Zeit aufkommenden Datenhelme, Helme und Equipment für das Erleben virtueller Realitäten, Cyberspace-Abenteuer, etc. ebenfalls eine Möglichkeit, diese schnell verteilbaren und optimal konservierten Mikrodüfte einzusetzen.

Im folgenden wird die Erfindung unter Bezugnahme auf die Figuren weiter erläutert. Es zeigen:
- Fig.1: eine Duft-Kompaktdiskette, bzw. eine Duft-CD,
- Fig.2a: einen Teilschnitt durch die Duft-Kompaktdiskette / Duft-CD aus Fig.1 mit Schlitzkanälen im nicht aktivierten Zustand,
- Fig.2b: einen Teilschnitt durch die Duft-Kompaktdiskette / Duft-CD aus Fig.1 mit Schlitzkanälen im aktivierten Zustand,
- Fig.3: einen Querschnitt durch verschiedene Variationen der Duft-CD aus Fig. 1,
- Fig.4a: einen Halbquerschnitt durch die Duft-Kompaktdiskette / Duft-CD aus Fig.1 im nicht aktivierten Zustand,
- Fig.4b: einen Halbquerschnitt durch die Duft-Kompaktdiskette / Duft-CD aus Fig.1 im teilweise aktivierten Zustand,
- Fig.4c: einen Teilquerschnitt durch die Duft-Kompaktdiskette / Duft-CD aus Fig.1 im voll aktivierten Zustand,
- Fig.5: eine Explosionszeichnung der Duft-CD aus Fig.1 mit verschiedenen Funktionsschichten,
- Fig.6: eine weitere Ausführungsform einer Duft-Kompaktdiskette mit Abspielbegrenzung und länderspezifischen Filtercodes, sowie eine teilweise Darstellung eines Abspielgeräts,
- Fig.7: eine weitere Ausführungsform der Duft-CD,
- Fig.7a: eine Variation der Ausführungsform nach Fig.7
- Fig.8: einen Teilquerschnitt durch eine weitere Ausführungsform einer Duft-Kompaktdiskette,
- Fig.9: einen unteren Diskettenrahmen einer weiteren Ausführungsform der Duft-CD,
- Fig.10: eine weitere Ausführungsform der Duft-CD mit manueller Vorbereitung der Aktivierungsmöglichkeit,
- Fig.11: einen Längsschnitt durch das Ausführungsbeispiel aus Fig. 10
- Fig. 12a: eine weitere Ausführungsform der Duft-CD mit Transportminimierung,
- Fig. 12b: die Ausführungsform der Duft-CD aus Fig.12a im aufgeklappten bzw. abspielbereiten Zustand,
- Fig. 13a: eine weitere Ausführungsform der Duft-CD mit Transportminimierung im Transportzustand,
- Fig.13b: die Ausführungsform der Duft-CD aus Fig. 13a im aktivierten Zustand,
- Fig. 14: eine weitere, einstückige Ausführungsform der Duft-CD als Klappdiskette.
- Fig. 15: einen schematischen Querschnitt durch ein Abspielgerät 24.

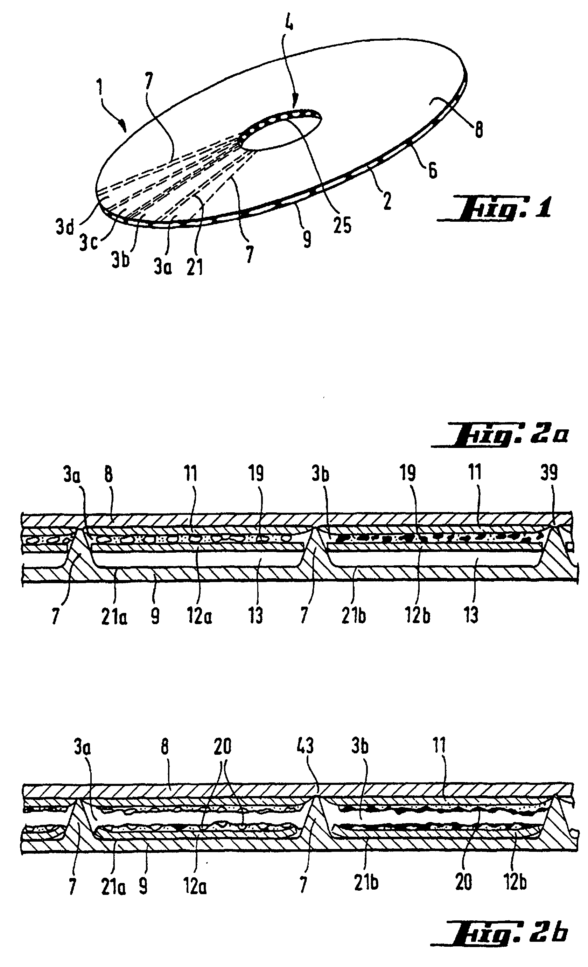

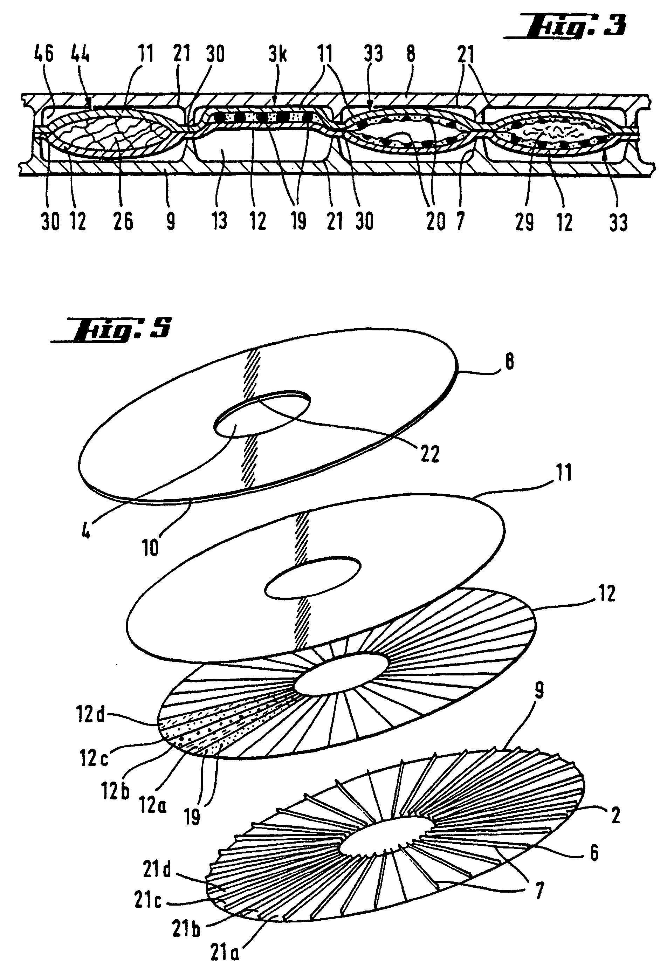

Das in Fig.1 und in Fig.2a bis Fig.5 dargestellte Ausführungsbeispiel der Erfindung zeigt eine flache Duft-Kompaktdiskette / Duft-CD (1), in welcher eine große Anzahl, (d.h., vorzugsweise zwischen 5 und 50) verschiedene Düfte auf kleinstem Raum innerhalb einer sehr flachen, kleinen Scheibe gelagert und dort zugleich perfekt konserviert werden.

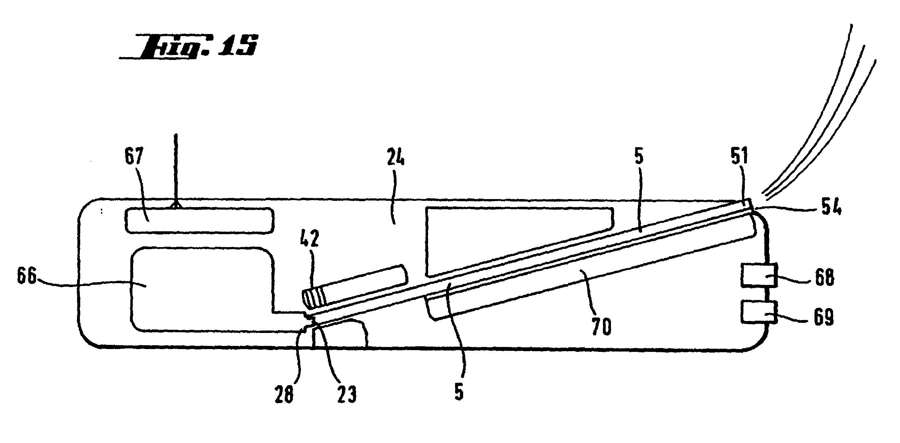

Derartige Duft-CD's dienen zunächst grundsätzlich dazu, um sie in kleinen Abspielgeräten (Fig.15) unabhängig von großen Maschinerien abspielen zu können, wie dies in der PCT/EP/92/02446 Fig.16 und 17 bereits für ein (noch nicht praktikables und noch vertikal durch den Duftträger abzuspielendes Duftmedium) beschrieben wurde. Die in der vorliegenden Erfindung vorgeschlagenen, extrem flachen Duft-CD's, die in kleinen, dezentralen Abspielgeräten vorgeführt werden können, machen es hierbei erstmals möglich, ereignisbezogene (z.B. Film-) Sets von vielen verschiedenen Düften zeitsynchron an jedem gewünschten Ort, in perfekter Qualität und zugleich individuell auf das persönliche Empfinden des Konsumenten oder Betrachters abgestimmt, abzuspielen.
Diese jeweils genau auf ein bestimmtes Ereignis oder Film abgestimmten Multi-Duftsets, die zugleich langfristig konservierbar sind, ermöglichen durch ihre kleine und bevorzugt flache Ausführung dabei auch erstmals eine sehr schnelle und kostengünstige Verteilung an ein dezentral verteiltes Massenpublikum, z.B. auf dem Postweg.

Bei einer derartigen Duft-Kompaktdiskette / Duft-CD (1), wird nun jeder einzelne, der verschiedenen Düfte zunächst in einem eigenen, extrem flachen Schlitzkanal 3a, 3b, 3c, etc. (Figuren 1, 2a, und 4a) gelagert, wobei jeder dieser Schlitzkanäle wiederum in einer weiteren, ebenfalls sehr flachen Schutzröhre 21 a, 21 b, etc., welche die verschiedenen Düfte in den Schlitzkanälen 3a, 3b, 3c, etc. luftdicht gegeneinander abschließen, untergebracht ist (Fig.2a). Diese Röhren sind in dem vorliegenden Ausführungsbeispiel radial auf der Duft-CD 1, angeordnet, (Fig.1).
Die zahlreichen Schutzröhren 21 a, 21 b, etc, welche um die duftführenden Schlitzkanäle 3a, b, c, etc. herum angeordnet sind, werden hierbei durch eine obere Diskettenhälfte 8, eine untere Diskettenhälfte 9, sowie seitliche Kanaltrennungen 6 in Form beidseitiger, länglicher Trennstege 7 in der Duft-Kompaktdiskette 1 gebildet (Fig.1 und 2a).

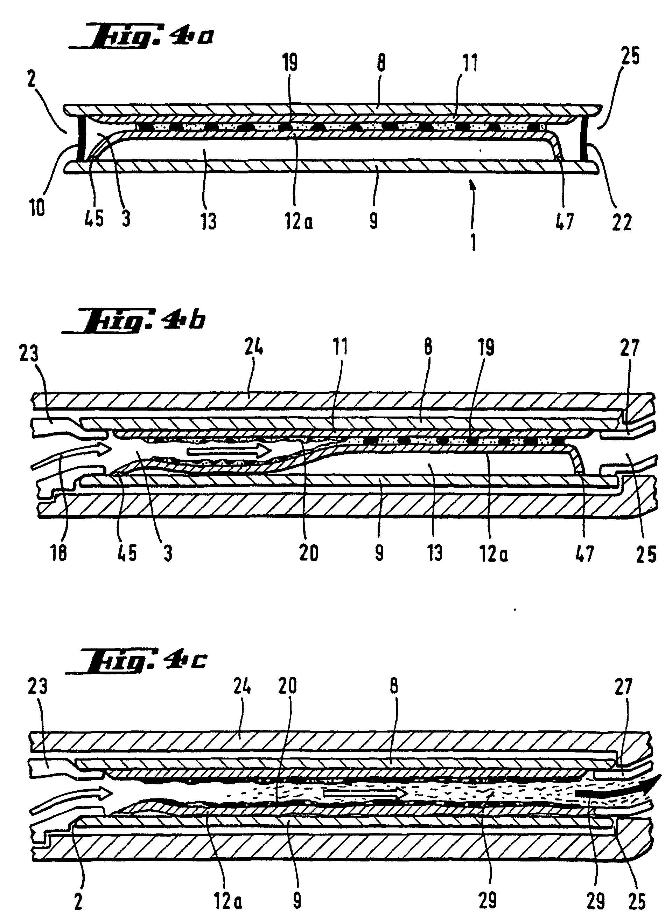

Die Schutzröhren 21 bilden hierdurch kleine, radial angeordnete Flachtunnel, in welchen die quer zur Duft-CD verlaufenden Schlitzkanäle 3 auch vor äußeren Umwelteinflüssen wie Wärme, Druck und Beschädigungen geschützt werden (Fig.2a). Diese Schutzfunktion wird insbesondere dadurch wichtig, daß die Schlitzkanäle 3 vorzugsweise druckempfindlich ausgeführt sind (Fign.2a und 4a), was weiter unten noch näher erläutert wird.
Um den auf der Duft-CD vorhandenen, knappen Platz optimal auszunutzen, verlaufen die Schlitzkanäle 3 des vorliegenden Ausführungsbeispiels vorzugsweise in konischer Form vom Rand der Diskette, wo sich jeweils der Einlaß 2 jedes Schlitzkanals 3 befindet, nach innen zu einem Auslaß-Panel 4.

Der Einlaß 2 und der Auslaß 25 der Schlitzkanäle 3 können hierbei zur zusätzlichen Haltbarmachung der Düfte mit Siegeln 10 und 22 versehen sein (Fig.4a), wobei diese Siegel geöffnet werden, sobald die Diskette in ein Abspielgerät gesteckt wird, bzw. erst dann, wenn sie auch tatsächlich von dem Abspielgerät abgespielt wird, (Fig.4b).
Bei der vorzugsweisen Art der Lagerung der Duftstoffe innerhalb der Schlitzkanäle 3 können diese Versiegelungen jedoch u.U. entfallen, da durch die vorliegende Ausführungsform der Diskette bereits eine langlebige Konservierung gewährleistet wird, wie im folgenden näher beschrieben wird.

Vorzugsweise werden hierbei die Duftstoffe an den Innenseiten der Schlitzkanäle 3 in Form mikroskopisch kleiner, sogenannter Mikroduft-Kapseln 19 aufgetragen (Fig.2a und 4a), wobei die Duftstoffe einerseits durch die Mikro-Verkapselung selbst, vollständig verschlossen sind, was durch den Ausschluß von Sauerstoff bereits eine sehr gute Konservierung auf kleinstem Raum gewährleistet. (Die Mikroverkapselung kleinster Mengen in flüssiger und in anderer Form vorliegender Stoffe, ist ein in der Druckindustrie seit langem bekanntes Verfahren.)
Desweiteren ergibt sich dadurch, daß die Schlitzkanäle 3, worin die verkapselten Duftstoffe gelagert sind, selbst noch ein weiteres Mal von einer schützenden und abschließenden Röhre, der Schutzhülle 21 ummantelt sind, eine weitere Konservierung der Duftstoffe.

Die Schutzhülle 21 hat hierbei auch die Funktion, die in den Schlitzkanälen 3 befindlichen Mikroduftkapseln 19 vor der Einwirkung durch mechanischen und anderen Druck, sowie Licht, Wärme, Feuchtigkeit und anderen Einflüssen zu schützen, da die Mikroduftkapseln relativ leicht vorzeitig geöffnet werden können, soweit ein Schlitzkanal 3 z.B. zusammengedrückt wird.

Dies spielt insbesondere auch beim Versand der Duftdisketten mit seinen z.T. vielfältigen, mechanischen und anderen Beanspruchungen eine wesentliche Rolle, da der Duft aus vorzeitig geöffneten Mikroduftkapseln 19 auch frühzeitig anfangen würde, zu altern und zu oxydieren, wobei sich der Dufteindruck z.T. erheblich verschlechtern kann.

Jeder einzelne Duftstoff ist in dem vorliegenden Ausführungsbeispiel somit dreifach versiegelt und konserviert:
1.) in der Schutzhülle 21
   (Schutz vor mechanischem Druck, Sauerstoff und Vermischung)
2.) in den Schlitzkanälen 3 (Schutz vor Vermischung und Oxydation)
3.) in den Mikroduft-Kapseln 19 (Schutz vor Oxydation)
womit auf kleinstem Raum (Duft-CD) eine sehr zuverlässige Konservierung und Aufbewahrung vieler verschiedener (z.B. 40) Düfte realisiert werden kann.

Für die szenengenaue Duftbegleitung eines TV-Spielfilms oder sonstigen Ereignisses wird die Duft-CD nun in ein entsprechendes Abspielgerät gelegt, wobei die Funktionsweise derartiger Abspielgeräte im wesentlichen bereits in der PCT/EP92/02446 des Anmelders beschrieben ist. Dadurch, daß im Unterschied zu dieser früheren Anmeldung das Transportmedium, z.B. Luft in der vorliegenden Konstruktion nicht auf dem kurzen Weg - quer durch den Duftträger - fließt, sondern längs durch die flach in der Diskettenscheibe liegenden Schlitzkanäle 3, sind hier die Fließwege um ein vielfaches länger. Hierdurch wird bei sehr geringem Platzbedarf des Abspielmediums die Oberfläche der einzelnen Düfte entscheidend vergrößert, was sowohl eine genauere Darstellung von Düften, als auch deren Abspielung in verschiedenen Stärkegraden möglich macht.

Andererseits wird mit den längeren Fließwegen in der extrem flach konstruierten Duft-CD der für den Abspielbetrieb notwendige Druck wesentlich höher, weshalb als Pumpantrieb eines entsprechenden Abspielgerätes für Duft-Kompaktdisketten kein Tangentialgebläse, wie in der früheren Anmeldung beschrieben, sondern eine der bekannten Arten von Pumpen für Luft und gasförmige Medien, wie z.B. eine normale Aquarium-Pumpe, bzw. Membranpumpe 66 verwendet wird (Fig.15).
Neben weiteren geeigneten Pumpentypen, wie Verdichterpumpen können hierfür auch sehr kleine, kompressorartige oder turbinenartige Pumpen verwendet werden.

Bei der film- oder musikbezogenen Vorführung dieser Düfte wird nun erstmals auch eine Technik realisiert, bei welcher der Trägerstoff (z.B. Luft) den verschlossenen und konservierten Duftstoff selbsttätig aus dem verschlossenen Zustand heraus öffnet, nach und nach aus den Versiegelungen der Konservierung herauslöst und den Duft anschließend mit sich führt und zum Konsumenten transportiert.
Das Trägermedium öffnet hier gewissermaßen die Duft-Flasche selbst und reichert sich dann selbsttätig mit den zu einer bestimmten Filmszene gehörigen Duftstoffen an.
Die Duftstoffe verbleiben bei dieser Technik bis zum unmittelbaren Zeitpunkt der Vorführung unter Luftabschluß, so daß trotz Unterbringung auf kleinstem Raum vorzeitige Alterungsprozesse zuverlässig vermieden werden.

Hierbei wird für die zu einem Spielfilm gewünschte, szenengenaue Entfaltung und Abgabe von Duftstoffen zunächst eine genau zu den Ereignissen dieses Films passend hergestellte Duft-CD 1 in ein entsprechendes Abspielgerät gelegt.
Hierbei wird innerhalb des Abspielgerätes 24 automatisch eine kleine Eingangsdüse 23 in den Schlitzkanal-Einlaß 2 des als erstes abzuspielenden Duftes hineingeschoben, während eine weitere Ausgangsdüse 27 in den auf der Duft-CD 1 innen gelegenen Auslaß 25 des Schlitzkanals 3 geschoben wird (Fig.4b).

Sobald während des Ablaufs eines Spielfilms, etc. nun der entsprechende, zu einer bestimmten Szene gewünschte Duft eingespielt werden soll, wird zusammen mit dem Film ein entsprechendes Signal, welches das Abspielgerät, z.B. durch einen entsprechenden Signalempfänger 67 (Fig.15), identifizieren kann, gespielt bzw. gesendet.
Hierdurch wird das vorgesehene, gasförmige Transportmedium, vorzugsweise Luft, innerhalb des Abspielgerätes 24 über eine (nicht dargestellte) Pumpe aktiviert und über die Eingangsdüse 23 in den Einlaß 2 des zum ersten gewünschten Szenenduft A zugehörigen Schlitzkanals 3a gepumpt.
Durch den sich dabei aufbauenden Druck werden nun zunächst die Wände am Einlaß 2 des Schlitzkanals 3a, welcher zwecks einfacher Herstellung vorzugsweise aus einer duftführenden Folienoberseite 11 und einer duftführenden Trennfolie 12a, (Fig.5), besteht, radial zur Fließachse auseinandergepresst (Fig.4b).

Damit sich der Luftstrom hierbei bis hin zum Ende des Schlitzkanals 3 fortsetzt und nicht seitlich entweichen kann, sind vorzugsweise bestimmte Teile der Folienseiten 11 und 12 des Schlitzkanals 3 miteinander und zum Teil auch mit den Diskettenhälften 8 und 9 verbunden.
So können die Folien 11 und 12 beidseitig neben jedem Schlitzkanal 3 jeweils mit einer seitlichen Folienverbindung 30 verbunden sein, welche sich kurz nach den duftführenden Teilen der Folien trennen.

Bei einer modifizierten Form des Schlitzkanals 3k (Fig.3) können diese seitlichen Verbindungen 30 zwischen den Folien 11 und 12 auch bestehen bleiben, wobei sich mit der Aktivierung dieses modifzierten Schlitzkanals 3k bevorzugt ein kleiner, duftführender Flachkanal 33 ergibt, der während des Abspielvorgangs als Duftkanal bestehen bleibt, ohne daß sich hierbei die Verbindungen 30 lösen, (siehe in Fig.3 die drei rechten Kanäle).

Eine derartige, seitliche Verbindung 30 neben den Schlitzkanälen 3 kann beispielsweise als Klebe-, Heißklebe-, Falz-, Falt- oder Stanzverbindung zustande kommen, oder auch dadurch, daß die Trennstege 7 der unteren Diskettenhälfte 9 so auf die obere Diskettenhälfte 8 auftreffen, daß bereits hierdurch die seitlichen Verbindungen 30 zwischen den Folien 11 und 12 geschaffen werden (etwa wie in Fig. 3).
Hierbei können sich auf der oberen Diskettenhälfte 8 ebenfalls obere Trennstege 46 befinden, welche mit der unteren Diskettenhälfte 9 oder deren Trennstegen 7 zusammenwirken, um hierbei die Abtrennungen der einzelnen Duftkanäle einzurichten.

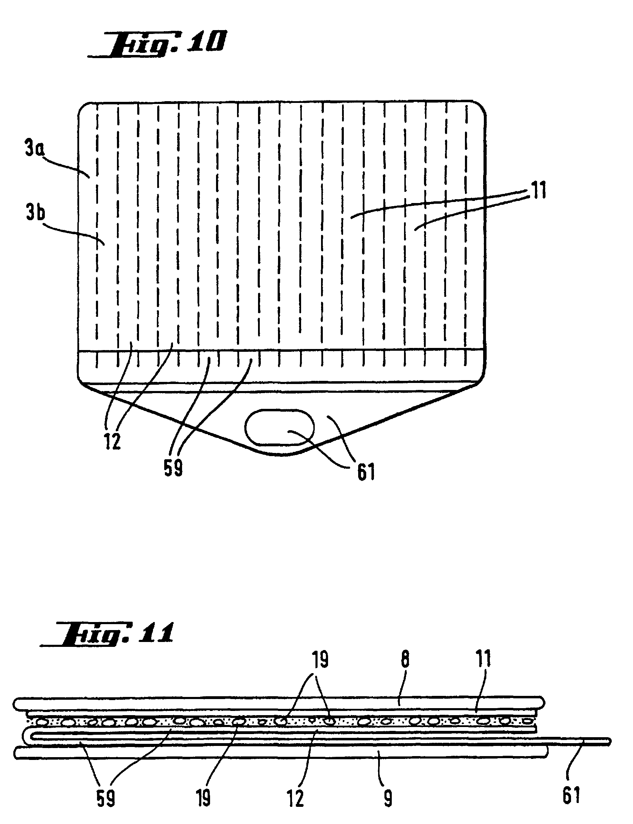

In einer leichten Abwandlung des in Fig.3 dargestellten Schlitzkanals 3k (zweite Schutzröhre 21 von links), kann dieser noch mit weiterem, eventuell leicht eingefaltetem Material versehen sein, (Fig.8) so daß sich der Schlitzkanal 3k bei einem Hindurchströmen von Luft ganz an die Wände des Schutzkanals 21 legt und somit ein größeres Durchflußvolumen als bei der Bildung eines Flachkanals 33 (wie in den beiden rechten Schutzröhren 21 in Fig.3) erreicht (Fig.8).

In einer weiteren Abwandlung des in Fig.3 dargestellten Schlitzkanals 3k wird dieser nicht mehr aus zwei Folien 11 und 12, sondern einstückig ausgebildet, wobei die Duftstoffe während des Herstellungsprozesses an den Wänden dieses einstückigen Flachkanals aufgetragen werden. Hiernach kann der Kanal jedoch ebenfalls flach gefaltet, gepreßt, oder geknickt werden und anschließend in einer Schutzröhre 21 untergebracht werden.
Derartige Flachkanäle können hierbei ebenfalls an den Enden mit siegeln 10 und 22, oder mit schließenden Perforationen, Klebungen, Falzungen, etc. versehen sein, welche beim ersten Durchströmen von Trägermaterial geöffnet werden.
Hierbei ist es auch möglich, derartige Flachkanäle während des Herstellungsprozesses so einzurichten, daß die Wände dieser Kanäle teilweise oder vollständig oder nur an den Enden unter einer inneren Spannung stehen. Soweit nun Luft in derartige Kanäle fließt und darin einen gewissen Druck aufbaut, springen die unter leichter Spannung stehenden Bereiche dieser Kanäle so auf, daß hierbei der Durchfluß von Luft und Duftstoffen, etc. vollständig ermöglicht wird.

Zur Stabilisierung des Luftstroms sind in den Fign. 4a - c vorzugsweise auch die duftführende Folienoberseite 11 mit der oberen Diskettenhälfte 8 verbunden und von den unteren Trennfolien 12 ist zumindest das vordere Ende mit einer Verbindung 45 und das hintere Ende durch eine weitere Verbindung 47 mit der unteren Diskettenhälfte 9 verknüpft Fig.4b).

Der sich nun weiter aufbauende Druck am Schlitzkanal-Einlaß 2 (Fig.4b) setzt sich dabei schließlich zum Inneren des Schlitzkanals 3a hin fort, (Fig.4c) wobei die Folienoberseite 11 und die duftführende Trennfolie 12a der Länge nach bis zum Auslaß 25 hin voneinander gelöst werden und die Trennfolie 12a in den Verdrängungs-Hohlraum 13 hinein gedrückt wird (Fign.4a-c und 2b).
Soweit hierbei die Folienoberseite 11 und die Trennfolie 12 am Rand eines Schlitzkanals 3k jeweils mit einer Verbindung 30 verbunden sind (wie in Fig.3), löst sich die Trennfolie 12 nur im mittleren Bereich von der Folienoberseite und bildet dabei dann den Flachkanal 33 (in Fig.3 die drei rechten Kanäle).

Hierbei werden die mit den Folien 11 und 12 verbundenen Mikroduft-Kapseln 19 auseinandergerissen und geöffnet, wobei die bis dahin noch verschlossenen Duftstoffe freigesetzt und ab nun von der weiteren, eingepressten Luft mitgeführt werden (Fig.4c und 2b). Soweit sich die Folien 11 und 12 eines Schlitzkanals 3 voneinander gelöst haben und die zuvor in den Mikroduft-Kapseln verschlossenen Duftstoffe aus ihrer Konservierung freigeben, wird der nun mit Duftstoffen angereicherte Luftstrom 29 anschließend durch die Schutzröhren 21a - x weitergeführt und über den Ausgang 14 zur Ausgangsdüse 27 weitergeleitet (Fig.4c).

Die nun mit den zu einer bestimmten Szene gehörenden Duftstoffen angereicherte Luft 29, wird nach dem Verlassen des Schlitzkanals 3a der Duft-CD 1 weiter aus dem Abspielgerät hinaus und zum Zuschauer hin befördert, so daß es z.B. in einer Filmszene, in der eine Orange zu sehen ist, auch entsprechend nach Orange duftet (Fig. 15).

Die Abspielung des nächsten, zu einer weiteren Szene gehörenden Duftes geschieht nun, indem die Duft-CD 1 innerhalb des Abspielgerätes 24 über entsprechende, weitere Signale so angesteuert und bewegt wird, daß der entsprechende Duft ebenfalls angespielt werden kann, wobei die beiden Ein- und Ausgangsdüsen 23 und 27 kurzfristig von der Duft-CD abgehoben werden und dann wieder in den Schlitzkanal 3b bzw. die Schutzröhre 21b des weiteren Duftes eingesetzt werden.
Der Durchfluß von Luft innerhalb der Duft-CD, kann bei einer Variation des vorliegenden Ausführungsbeipiels auch umgekehrt von innen nach außen statt von außen nach innen erfolgen, wobei die Eingangsdüse 23 zur Ausgangsdüse 27 wird und umgekehrt. Die angereicherte Luft wird dann ebenfalls von dem jeweils anderen Duftausgang zum Zuschauer geleitet.

Um auf einer einzigen Duft-CD alle 30, 50 oder mehr für einen Film, ein Musikstück, etc. benötigten Düfte tatsächlich unterbringen zu können, ist es desweiteren vorteilhaft, die jeweilige Fläche, die für einen Duft benötigt wird, durch bestimmte, konstruktive Maßnahmen möglichst klein zu halten.

Die Fläche, welche benötigt wird, um einen verkapselten Duft insgesamt auf einer Duft-CD unterbringen zu können, läßt sich zunächst dadurch beeinflussen, wie dicht die Duftkapseln in den duftführenden Schichten der Duft-CD aufgetragen werden und wie groß die einzelnen Kapseln sind. Desweiteren kann auch die Intensität der Duftmischung, welche als Grundlage des Verkapselungs-Prozesses gewählt wird, die benötigte Duftfläche beeinflussen.
Um auf einer Duft-CD möglichst viele Düfte unterbringen zu können, werden bei den vorliegenden Duft-CD's daher vorzugsweise besonders intensive Mischungen der zu verkapselnden Düfte und zugleich möglichst viele Duftkapseln pro Flächeneinheit aufgetragen, so daß jeweils nur wenig Fläche pro Duft benötigt wird.

Zur Einhaltung maximaler Szenengenauigkeit und Vermeidung jeglicher Duftüberlagerungen können in dem vorliegenden Abspielgerät ebenfalls die in der früheren Anmeldung PCT/EP92/02446 (Fig.16 u. 17) für zentrale Abspielgeräte vorgeschlagenen (und hierbei im Unterschied zu allen bisherigen Vorschlägen erstmals funktionierenden) Maßnahmen auf ein dezentrales Abspielgerät angewendet werden, d.h. äußerste Minimalisierung der verwendeten Luftmengen, Präzisierung des Duftverlaufs und Minimalisierung der verwendeten Leitungen.

Die durch das Abspielgerät von der Duft-CD abgerufenen Duftmengen bewegen sich daher nur auf einem extrem niedrigen Niveau, d.h. diese liegen vorzugsweise bei einem Tausendstel bis Zehntausendstel der Leistungsmenge einer Klimaanlage und werden darüber hinaus relativ gebündelt zum Zuschauer geleitet, wie ebenfalls in der PCT/EP92/02446 vorgeschlagen. Die Düfte sind daher nur in einem sehr kleinen Raumvolumen um die Nase eines Zuschauers herum wahrnehmbar, was es ermöglicht, mit besonders geringen Duftmengen zu arbeiten.
Hierdurch wird neben den bereits vorgeschlagenen Maßnahmen, ebenfalls eine Unterbringung der Düfte auf sehr kleinem Raum innerhalb der Duft-CD ermöglicht.

Bei einem nicht dargestellten Ausführungsbeispiel des dezentral gesteuerten Abspielgerätes werden hierbei vorzugsweise nur zwischen 0,0001 und 0,5 Liter bedufteter Luft pro Sekunde verwendet. Bei einem anderen, bevorzugten Ausführungsbeispiel des dezentralen Abspielgerätes werden hierbei zwischen 0,003 und 0,3 Liter pro Sekunde verwendet und bei einem besonders bevorzugten Ausführungsbeispiel der Erfindung werden hierbei zwischen 0,01 und 0,2 Liter pro Sekunde verwendet.

Hierdurch wird es ebenfalls möglich, eine Duft-Vorführung sehr präzise in ihrem Ablauf zu gestalten, da sich derartig kleine Duftmengen sehr schnell verflüchtigen und so eine szenengenaue Darbietung ermöglichen.

Durch die, im wesentlichen nur im Bereich der Nase eines einzelnen Zuschauers wahrnehmbaren Dufteindrücke, wird es ebenfalls möglich, diese kleinen Duftmengen individuell für diesen Zuschauer, bzw. für jeden Zuschauer anders, einstellbar zu machen.

Diese Einstellbarkeit der abgegebenen Duftmengen wird insbesondere dadurch wichtig, da die Geruchsempfindlichkeit der Nasen der Konsumenten u. U. sehr verschieden ist.
Die Duftvorführungen können bei einem bevorzugten Abspielgerät entsprechend individuell in ihrer Stärke eingestellt werden, so daß jeder Verbraucher die von ihm bevorzugte Intensität der Vorführung genießen kann. Desweiteren ist es bei aufwendigeren Ausführungsformen des Abspielgerätes ebenso möglich, die Temperatur, Menge und Duftzyklus-Dauer der vorgeführten Düfte individuell zu beeinflussen.

Sofern der Konsument sich zwar für duftbegleitete Film-Vorführungen begeistert, aber eine spezifische Abneigung gegen ganz bestimmte Düfte hat, ist es bei dem vorliegenden Abspielgerät weiterhin möglich, diese Düfte in Klassen einzuteilen, wobei der Benutzer die von ihm nicht gewünschten Duftklassen quasi "abwählen" kann. Die Steuersignale für diese nicht gewünschten Düfte werden dann von dem Abspielgerät einfach ignoriert und die entsprechenden Düfte werden übersprungen.
Die entsprechende, individuelle Einrichtung einer Duftvorführung wird weiter unten, bei der Beschreibung der Fig.6 dargestellt.

Zum Zwecke einer möglichst einfachen Produzierbarkeit wird die Duft-CD 1 während des Herstellungsprozesses vorzugsweise aus vier Funktions-Schichten zusammengesetzt und zwar aus der oberen Diskettenhälfte 8, der duftführenden Folienoberseite 11, der duftführenden Trennfolie 12 und der i.d.R. mit Trennstegen 7 versehenen, unteren Diskettenhälfte 9 (Fig.5). Während der Herstellung werden die einzelnen Funktionsschichten 8, 9, 11 und 12 nun zusammengepreßt und z.T. -geschweißt (z.B. mit Ultraschall), wobei sich durch die Beschaffenheit und Formgebung der Einzelschichten (Fig.5 und Fig.1) nun die einzelnen, vorgesehenen und weiter oben ausgeführten Funktionsmerkmale der Duft-CD ergeben. Hierbei entstehen die Schlitzkanäle 3 vorzugsweise aus dem Zusammenpressen und z.T. - kleben der Schichten 11 und 12, wobei die Segmentierung der Schlitzkanäle 3a, 3b, etc. bzw. der Trennfolien 12a, 12b, etc. durch das Auftreffen der Trennstege 7, die sich vorzugsweise auf der unteren Diskettenhälfte 9 befinden, auf die obere Diskettenhälfte 8 entstehen.

Um zur Erzeugung kleiner, luftdruckfester Schutzröhren 21a - x jeweils eine feste Verbindung zwischen der unteren Diskettenhälfte 9 bzw. den darauf befindlichen Trennstegen 7 und der oberen Diskettenhälfte 8 zu erreichen, können die Spitzen der Trennstege 7 mit verschiedenen Verfahren, (etwa mit Ultraschall-Schweißverbindungen 39, wie in Fig.2a, oder Steck-, Klebeund sonstigen Verknüpfungen) mit der oberen Diskettenhälfte 8 verbunden werden.
Hierdurch können die, zuvor durch eine Art Druckverfahren auf zumindest eine der Folien aufgebrachten, verschiedenen Duftbahnen der Folien 11 und 12 (Fig.5) auch jeweils segmentiert werden. Eine derartige, vorzugsweise luftdruckfeste Segmentierung ist insofern vorteilhaft, weil dadurch vermieden wird, daß der Luftstrom während der Aktivierung eines Schlitzkanals 3 noch in einen Nachbarkanal hinein fließt und dadurch eventuell einen unerwünschten Mischduft erzeugen könnte.

Die Verbindungen der Trennstege 7 und der anderen Diskettenhälfte können hierbei auch durch die duftführenden Folien 11 und 12 hindurch entstehen z.B. als Steck- oder Klebeverbindung.

Bei einer Steckverbindung werden die Folien am Rand jeweils abgeklemmt, während die bei einer Ultraschall-Schweißverbindung 39 z.B. aus Kunststoff bestehenden Folien im Bereich der auftreffenden Trennstege 7 kurz erhitzt werden und dabei zusammengeschweißt werden. Eine Klebeverbindung bei z.B. aus Zellulose bestehenden Folien kann etwa zustande kommen, indem der Klebstoff kapillarisch oder durch kleine Perforationslöcher durch die Folien zur andern Diskettenhälfte hindurchdringen und die Duftbahnen hierbei segmentieren.

Bei einer weiteren Abwandlung des Herstellungsverfahrens der Duft-CD werden duftführende Trennfolien 12a-x nach dem Auftragen der Düfte zunächst ebenfalls als zusammenhängende Folie 12 belassen, (Fig.5), wobei die einzelnen Düfte hier ebenfalls auf die Gesamtfolie 12 aufgetragen oder gedruckt werden, ohne diese in einzelne Trennfolien 12a, 12b, etc. zu zerteilen.
Durch leichte Schnitte in die Folienoberfläche (eine Art Sollbruchstellen) werden nun vorzugsweise einzelne Duftlamellen vorbezeichnet. Desweiteren wird hier auf die Verwendung einer duftführenden Folienoberseite 11 verzichtet und die Trennfolie 12 gleich mit den verschiedenen Mikroduft-Kapseln 19 der einzelnen Duftbahnen auf die obere Diskettenhälfte 8 geklebt.
Beim Zusammenfügen bzw. -pressen der unteren 9 und oberen Diskettenhälfte 8, auf welche bereits die vorsegmentierte Trennfolie 12 mit allen benötigten Düften geklebt ist, treffen nun die Trennstege 7 der unteren Diskettenhälfte so mit der oberen Hälfte zusammen, daß dabei die duftführenden Trennfolien 12 in die Einzelfoiien 12a, 12b, usw. aufgeteilt werden. Hierbei werden die duftführenden Schlitzkanäle 3a - 3x schließlich zwischen den Trennfolien 12 und der oberen Diskettenhälfte 8 gebildet und durch die Trennstege 7 luftdicht voneinander abgeteilt.

Bei weiteren, nicht dargestellten Ausführungsbeispielen der Erfindung sind vielfältige Kombinationen der Bestandteile einzelner Ausführungsbeispiele der Duft-CD, sowie Variationen und Abwandlungen der beschriebenen Herstellungsprozesse zur Erzeugung ähnlich funktionierender Duftdisketten denkbar.

Die Düfte, welche schließlich durch das vorliegende Abspielgerät vorgeführt werden, können zur Perfektionierung des Dufteindruckes außerdem, in ihrer Darbietungsform modifiziert werden.
Eine wesentliche Verbesserung des Dufteindrucks kann in bestimmten Fällen z.B. erreicht werden, wenn diese vor, bzw. bei der Abgabe an den Zuschauer zusätzlich durch einen kleines (nicht dargestelltes) Heizelement aufgewärmt, bzw. erhitzt werden, um beispielsweise den typischen Dufteindruck eines heißen Kaffees noch perfekter darzubieten.

Eine derartige Erhitzung kann vorzugsweise auch dadurch erreicht werden, daß die zu erwärmenden Düfte, bevor diese zum Zuschauer gelangen, durch ein umlenkendes Ventil in einen weiteren Kanal zu einem entsprechenden, bereits vorgewärmten Mikroheizelement umgeleitet werden. Ein derartiges Verfahren ist vor allem dann vorteilhaft, wenn die im Abspielgerät verwendeten Heizelemente zu träge sind, um in einem filmischen Ablauf sehr schnell aufgeheizt und dann beim nächsten Duft u.U. sehr kurzfristig wieder abgekühlt werden zu können.

Bei dem vorliegenden dezentralen Abspielgerät (nicht dargestellt) kann es umgekehrt auch zusätzlich möglich gemacht werden, die zum Zuschauer hinströmenden Duftstoffe zu kühlen, um hierdurch ebenfalls den Dufteindruck z.B. durch ein Eis oder durch eine kühle Herbstbrise zu imitieren.

In etwas aufwendigeren Abspielgeräten (nicht dargestellt) kann eine weitere Verbesserung der durch das Abspielgerät von der Diskette abgerufenen Düfte, bei bestimmten Aromen und Duftstoffen auch erreicht werden, wenn diese vor der Abgabe an den Zuschauer mit etwas Feuchtigkeit angereichert werden.
So kann etwa der Dufteindruck einer vom Regen feuchten Wiese perfektioniert werden, wenn der relativ trocken gelagerte und von normaler Luft abgerufene Wiesenduft anschließend durch einen (nicht dargestellten) Moisturizer geleitet wird.
Entsprechend kann auch das abrufende Medium selbst, etwa Luft vor dem Hindurchströmen durch die Diskette mit Feuchtigkeit angereichert werden.

Ein derartiger Moisturizer kann sich auch auf der Diskette selbst befinden, so daß z.B. das Trägermedium vor oder nach dem Eindringen in die duftführenden Kanäle durch eine entsprechende Feucht-Mikrokammer (nicht dargestellt), z.B. durch eine Art permeables moisturing Pad geleitet werden.

Ein derartiges Anfeuchten der bedufteten Luft kann auf der Diskette u.U. auch durch mikroverkapselte Feuchtigkeit erreicht werden, welche dann auf ähnlichem Weg freigesetzt wird, wie die weiter oben beschriebene Freisetzung von Düften aus Mikrokapseln.

Sofern mit dem vorliegenden Abspielgerät eine starke, vertikale Ausrichtung der ausströmenden und mit Duftstoffen angereicherten Luft angestrebt wird, kann das Trägermedium z.B. Luft auch bei einem dezentralen Abspielgerät mit bestimmten Mengen von Helium gemischt werden, (was für den Menschen absolut ungefährlich ist, da Taucher und sogar Asthmatiker hierdurch besser atmen können). Das Helium kann hierbei in kleinen Kartuschen, ähnlich den CO₂ Kartuschen in Syphonflaschen, innerhalb des Abspielgeräts untergebracht sein (nicht dargestellt).
Hierbei ist es z.B. auch möglich, daß zunächst die Düfte selbst durch einen Mikrostrom aus Helium aus der Kartusche abgerufen werden. Da, wie weiter oben beschrieben, die Fläche bzw. der Raum in dem die Düfte in der Diskette untergebracht werden, durch verschiedene Maßnahmen, z.B. Intensivierung der Duftausgangsbasis, vorzugsweise sehr klein gehalten wird, ist es hierbei möglich, für das eigentliche Abrufen des Duftes nur sehr geringe Mengen an Helium zu verwenden.
Dieses Gemisch aus kleinsten Mengen an Helium und intensivem Duft wird dann im Anschluß mit einem etwas größeren Luftstrom vermischt, um es auf die normale Duftintensität zu verdünnen und wird dann schließlich zum Zuschauer hin weitertransportiert.
Dieser verdünnende Luftstrom kann auch, wie weiter oben beschrieben, zuvor noch mit Feuchtigkeit versehen werden, oder zusätzlich auch aufgeheizt werden.
Kartuschen die nur für das Abrufen von Düften verwendet werden, können auch mit anderen Stoffen als Helium gefüllt werden.

Bei einem weiteren Ausführungsbeispiel der Duft-Kompaktdiskette 1, welches in Fig.3 in dem ersten, linken Kanal dargestellt ist, werden in den Schlitzkanälen 3 keine Mikroduft-Kapseln 19 untergebracht, sondern andere und ebenfalls extrem flach angeordnete, dufttragende Materialien 26, wobei sich bei einem Einströmen unter Druck befindlicher Luftmassen die Folien 11 und 12 in diesem Falle nicht vollständig auseinanderbewegen, sondern um das entsprechende Material 26 herum als Hülse, bzw. als modifizierter Flachkanal 44 erhalten bleiben. Hierbei durchströmt die Luft nun das permeable oder semipermeable, dufttragende Material 26.
Die Schutzröhren 21 a, 21 b, etc. die sich um die modifizierter Flachkanäle 44 herum befinden und diese gegeneinander luftdicht verschließen (Fig.2a) werden am Einlaß 2 und Auslaß 25 in diesem Fall jedoch vorzugsweise mit Siegeln 10 und 22 versehen, da die Konservierungsmöglichkeiten dieser anderen Materialien 26 nicht so weit reichen dürften, wie bei Mikroduft-Kapseln. Als duftführende Materialien 26 sind in diesem Fall auch sehr dünne und flache Vliese verwendbar.
Diese Vliese werden hierbei vorzugsweise an dem Rand der modifizierten Flachkanäle 44 angebracht.

In einer Variation dieser Ausführungsform einer Duft-CD können auch verschiedene Mikro-Aufbewahrungsarten für Düfte innerhalb einer Duft-CD miteinander kombiniert werden, so wie dies in Fig.3 dargestellt ist. So können die Düfte hier in dem linken Kanal über dufttragende Materialien 26 und in anderen Kanälen über Mikroduft-Kapseln 19 aufbewahrt und konserviert werden.
Diese Ausführungsform empfiehlt sich insbesondere, sofern bei dem zu begleitenden Film einer der wenigen Düfte verlangt wird, bei denen eine Mikroverkapselung nur schwierig realisierbar ist, wie z.B. bei Kaffeeduft.
Somit können auf einer derartigen Duft-CD sowohl verkapselbare Düfte, als auch sonstige Düfte in anderen Aufbewahrungsformen in der optimal möglichen Weise aufbewahrt werden.

Durch die große Unterschiedlichkeit in der Zusammensetzung von Düften, sind für eine Aufbewahrung z.T. auch sehr unterschiedliche Verkapselungsverfahren notwendig. Daher wird für eine Duft-CD vorzugsweise jeder Duft mit dem für ihn optimalen Verkapselungsverfahren hergestellt und aufgetragen.

Soweit andere Verfahren in Frage kommen, die für die Aufbewahrung ganz bestimmter Düfte ein optimales Ergebnis erreichen, werden diese auf entsprechenden, weiteren Duftbahnen verwendet, so daß auf jeder Duftbahn jeweils das optimale Duft-Speicherverfahren zur Anwendung kommt.
So ist es beispielsweise durchführbar, empfindliche Düfte in Form eines Duft-Harzes, Duft-Gels oder weiteren Kombinationen von Düften und Trägerstoffen zu lagern. Soweit die entsprechenden Aufbewahrungsformen nicht mit einem Verkapselungsverfahren der Düfte verbunden sind, werden diese Duftbahnen zur besseren Konservierung am Ein- und Ausgang i.d.R. mit Versiegelungen 10 und 22 versehen, etwa wie dies in Fig.4a dargestellt ist.

Um die Unterschiedlichkeit in der Haltbarkeit von Duftstoffen, die einerseits verkapselt und andererseits mit anderen Verfahren gelagert werden, noch weiter zu verringern, sind neben den Versiegelungen 10 und 22 auch weitere Verfahren zur Konservierung anwendbar.

Hierbei werden auf der Diskette die duftführenden Bahnen der nicht verkapselten Düfte zusätzlich mit kleinen Mengen von Stoffen gefüllt, die eine vorzeitige Alterung und Oxydation verhindern. So können etwa die modifizierten Flachkanäle 44 (Fig.3) hiermit befüllt werden, oder, soweit etwa duftführende Vliese verwendet und direkt auf der Wand eines Schlitzkanals 3 angebracht sind, (etwa, wie die geöffneten Bahnen in Fig. 2a oder 4c dargestellt sind) können auch die Schlitzkanäle 3 selbst hiermit befüllt werden.

Hierfür kommen vorzugsweise gasförmige Stoffe in Frage, welche nach dem Einfüllen durch die Versiegelungen 10 und 22 innerhalb der entsprechenden Duftbahn festgehalten werden. Als derartige Oxydationshemmer 62 können z.B. sehr kleine Mengen an Helium oder Kohlendioxyd verwendet werden, die in die entsprechenden Duftbahnen der Diskette eingebracht werden. Als weitere Stoffe sind andere, gasförmige Stoffe denkbar, z.B. solche, die auch in der Haltbarmachung von Nahrungsmitteln und Getränken verwendet werden.

Als weiteres Verfahren ist es auch möglich, in der Diskette nach dem Befüllen mit Duftstoffen ein mehr oder weniger starkes Vakuum zu erzeugen, bevor die Versiegelungen 10 und 22 angebracht werden. Werden die Siegel 10 und 22 nun verschlossen, verbleibt in den verschlossenen Duftbahnen ein Vakuum, so daß an die empfindlichen Duftstoffe, die nicht verkapselt wurden, ebenfalls kein Sauerstoff oder andere, den Alterungsprozess beschleunigende Stoffe gelangen können.

Sobald die Siegel 10 und 22 beim Abspielen geöffnet werden, löst sich das Vakuum schließlich auf, worauf die Vorführung des entsprechenden Duftes beginnen kann. Werden stattdessen kleine Mengen von Oxydationshemmern verwendet, werden diese beim Abspielen der Diskette automatisch durch die duftführenden Stoffe z.B. Luft aus der Duftbahn gedrückt.

In einer weiteren, nicht dargestellten Ausführungsform derartiger Mischdisketten, welche Duftbahnen mit sowohl verkapselten als auch nicht verkapselten Duftstoffen aufweisen, können Disketten hergestellt werden, bei denen alle Duftbahnen in der Form der zuvor beschriebenen, nicht verkapselten Aufbewahrungsarten gelagert werden.
Auch wenn derartige Disketten trotz der beschriebenen, verschiedenen Konservierungsverfahren wahrscheinlich nicht die Haltbarkeit von Disketten mit durchweg verkapselten Düften aufweisen, können diese u.U. für den kurzfristig verwertbaren Bedarf verwendet werden.

Bei dem weiteren, in Fig.6 dargestellten, bevorzugten Ausführungsbeispiel einer Duft-CD 5 wird das u.U. aufwendige Drehen der S-CD für das Abspielen eines jeweils folgenden Duftes und dem damit verbundenen Abheben und wieder Einklinken der Ein- und Ausgangsdüsen 23 und 27 in den Ein- und Auslaß 2 und 25 des entsprechenden, neuen Duftes vollständig vermieden.
Zu diesem Zweck wird nun nach dem Einlegen der hier z.B. rechteckigen Duft-CD in das Abspielgerät ein Eingangsdüsen-Panel 28 in die Schlitzkanal-Einlässe 2 der Düfte eingeschoben, wobei für jeden einzelnen Duft eine eigene Eingangsdüse 23 mit einem eigenen Ansteuer-Ventil 48 vorhanden ist. Eventuell an den Einlässen 2 der duftführenden Schlitzkanäle 3a, b,etc. vorhandene Versiegelungen 10 werden bei dem Anschluß der Duft-CD an das Abspielgerät 24 durch die Eingangsdüsen 23 geöffnet. Für den beabsichtigten Wechsel von einem Duft auf einen anderen muß bei diesem Ausführungsbeispiel nur noch jeweils auf die neue Leitung zu der weiteren Eingangsdüse 23 umgeschaltet werden.

Eine derartige Art der Ansteuerung der Duft-Diskette dürfte zu einer wesentlichen Vereinfachung in der Herstellung und Funktion der Abspielgeräte führen, da nun fast keine beweglichen und u.U. anfälligen, mechanischen Teile mehr vorhanden sind (Fig.15).
Als Duftträger können hier, wie weiter oben beschrieben, bevorzugt ebenfalls Mikroduft-Kapseln 19 verwendet werden, die bei einem Einströmen von Luft 18 in den jeweiligen Schlitzkanal 3 durch das Ablösen von Trennfolien 12, u.ä.m. von selbst zerreißen und dadurch automatisch an den Luftstrom abgegeben werden.

Die nun mit Duftstoffen angereicherte Luft 29 fließt, durch die nachströmende Luft weitergeschoben, zu einem Ausrichter 51, welcher die verschiedenen Luftströme der einzelnen, jeweils aktivierten Duftkanäle 21 sammelt und zu einer Fokus-Ausgangsdüse 54 leitet. Die Fokus-Ausgangsdüse 54 hat im Betriebszustand schließlich eine Verbindung zum Abspielgerät, von wo die aktivierten Düfte über ein kurzes, weiteres Leitungsstück schließlich zum Zuschauer und/oder -hörer transportiert werden (Fig.6).
Der die Luftströme sammelnde Ausrichter 51 und die Fokus-Ausgangsdüse 54 sind hierbei bevorzugt Bestandteil der Duft-Diskette 5 selbst. Sofern sich in diesen Duftleitungen bei längerem Betrieb Ablagerungen von verschiedenen Düften ergeben sollten, was im Lauf der Zeit u.U. unerwünschte Überlagerungen mit Düften früherer Vorführungen nach sich ziehen könnte, hat dies den Vorteil, daß diese Duftleitungen mit jedem Wechsel einer Duft-CD erneuert werden (Fig.6).

Bei einer vorzugsweisen Abwandlung des beschriebenen Ausführungsbeispiels, wird der aus der Fokus-Ausgangsdüse 54 der Duft-CD 5 ausströmende Duftstrom, ohne weiteres Leitungsstück im Abspielgerät, direkt von der Duft-CD zum Zuschauer geleitet (Fig. 6 und Fig.15).
Hierdurch ist es schließlich möglich, daß die von dem Abspielgerät vorgeführten Duftstoffe, mit dem Abspielgerät selbst, in keiner Weise in Berührung kommen. Dies hat den Vorteil, daß sich auch bei sehr intensiver Nutzung eines solchen Abspielgerätes für Duft-CD's an keiner Stelle des Gerätes irgendwelche Ablagerungen von Duftstoffen im Laufe der Zeit ergeben können. Im Sinne einer wartungsfreien Handhabung solcher Geräte kann dies eine benutzerfreundliche Vereinfachung darstellen (Fig.15).
Die Duft-CD wird bei dieser Variante der Vorführ-Anordnung vorzugsweise nicht flach innerhalb des Abspielgeräts gelagert, sondern in einem schrägen Winkel, z.B. in einem 30° Winkel, so daß die in der Duft-CD befindlichen Duftstoffe ebenfalls in einem leicht schrägen Winkel zum Zuschauer ausströmen können, (Fig.15).

Sofern die Düfte in anderen, nicht dargestellten Ausführungsformen des Abspielgerätes 24 nach dem Verlassen der Duft-CD 5 dennoch ein größeres Stück Leitungen und ev. Apparaturen innerhalb des Abspielgerätes durchlaufen, könnten sich dort mit der Zeit u.U. weniger angenehm duftende Ablagerungen von Düften früherer Vorführungen ergeben. In diesem Fall wird für ein derartiges Abspielgerät eine spezielle, nicht dargestellte Reinigungsdiskette angefertigt, wobei in den Schutzröhren 21 statt duftführender Schlitzkanäle ein Vlies o.ä. mit einer Reinigungsflüssigkeit enthalten ist, welche beim Betrieb dann durch die entsprechenden, verunreinigten Leitungen des Abspielgerätes fließt und schließlich aufgefangen wird.

Soweit nun ein in der Duft-CD befindlicher Duft bei einer Vorführung gespielt wird (was in Fig.6 schematisch dargestellt wird) wird lediglich das Ventil 48 des entsprechenden Schlitzkanals 3 geöffnet. Die Tendenz eines hierbei möglicherweise über den Ausrichter 51 erfolgenden Rückstaus in andere, duftführende Schutzkanäle 21 oder Schlitzkanäle 3 hinein, wird dadurch weitgehend unterbunden, daß die Ventile 48 der anderen Düfte geschlossen bleiben und so kein rückwärtiger Luftfluss mit eventuellen Duftvermischungen stattfinden kann.
Ein anderer Schutz vor einem Rückstau kann hierbei dadurch gewährleistet werden, daß sich am Ende der duftführenden Schutzröhren 21 kurz vor deren Übergang in den Luftstrom-Ausrichter 51 kleine Auslaß-Ventile 56 befinden, die einen Luftstrom im wesentlichen nur in Richtung des Ausrichters 51 zulassen.

Da die Duftstoffe nach dem ersten Abspielen bereits mit Sauerstoff und weiteren Materialien in Berührung kommen, beginnt nach dem ersten, noch perfekten Abspielen der Düfte bereits deren Alterung.
Um zu verhindern, daß nun bei einem zweiten, eventuell sehr viel späteren Abspielen dieser Duft-Diskette die Düfte in stark gealterter und möglicherweise z.T. weniger angenehmer Form abgespielt werden, (was für das neue Medium keinen guten Ruf einbringen würde), werden die Duft-CD's aller beschriebenen Ausführungsformen vorzugsweise so gestaltet, daß diese nur begrenzt, bzw. nur einmal abgespielt werden können.

Diese begrenzte Abspielbarkeit kann bei dem in Fig.6 gezeigten Ausführungsbeispiel beispielsweise eingerichtet werden, indem innerhalb der Duft-CD 5 eine kleine Markierzunge 40 eingerichtet wird, die nur durch eine kleine Abtastöffnung 41 von außen zugänglich ist (Fig.6).
Wird das Abspielgerät nun aufgefordert, den Abspielvorgang in Betrieb zu setzen, wird zunächst ein im Abspielgerät 24 befindlicher Stift 42 (Fig.6), der genau durch die Abtastöffnung 41 paßt, in die Duft-CD 5 hineingeschoben. Erkennt nun der Abtaststift 42, daß die Markierzunge 40 noch vorhanden ist, wird die Duft-CD für den Abspielvorgang freigegeben.

Nach dem Beginn des Abspielvorganges wird der Abtaststift 42 schließlich noch ein Stück weiter in die Duft-CD 5 eingeführt, wobei die Markierzunge 40 umgeknickt oder zerstört wird. Hierdurch wird schließlich dokumentiert, daß die Duft-CD bereits einmal gespielt worden ist (Fig. 6 und Fig. 15).
Erkennt der Abtaststift vor dem Abspielvorgang andererseits, daß die Duft-CD 5 schon einmal abgespielt worden ist und sich die Düfte durch einen möglicherweise fortgeschrittenen Alterungsprozeß bereits negativ verändert haben, wird der Abspielvorgang nicht in Betrieb gesetzt.

Die Zerstörung der Markierzunge 40 muß hierbei nicht schon nach dem ersten, abgespielten Duft erfolgen, sondern wird vorzugsweise erst nach einem späteren, z.B. dem vorletzten Duft eines Spielfilms eingeleitet, um zu ermöglichen, daß ein nur halb gesehener Film auch später noch mit Duftbegleitung zu Ende gesehen werden kann.

Sofern beabsichtigt ist, die Duft-CD mehr als einmal abspielbar zu machen, kann die Freigabe eines weiteren Abspielvorganges auch an ein zwei- oder mehrmaliges zuvor erfolgtes Abspielen gekoppelt werden. Hierbei können z.B. entsprechend mehrere Markierzungen 40 hintereinander vorhanden sein oder nebeneinander, wobei diese dann durch entsprechend modifizierte Abtaststifte lokalisiert werden.
Statt auf mechanischem Wege ist eine Erkennung und Steuerung der Abspielhäufigkeit einer Diskette natürlich auch auf elektronischem oder anderen Wegen durchführbar. So ist es etwa machbar, diese Informationen auf einem auf der Diskette befindlichen Magnetstreifen 50 (Fig.6) zu realisieren, welcher durch das Abspielgerät 24 über einen entsprechenden Magentstreifen-Leser 70 gelesen werden kann (Fig.15).

Da ein wichtiger Alterungsfaktor einer Duftdiskette auch dadurch bedingt ist, wieviel Zeit verstrichen ist, seit sie das erste Mal abgespielt wurde, kann die Abspielbegrenzung auch durch einen bestimmten Zeitraum vordefiniert werden, welcher als Information auf der Diskette und/oder dem Abspielgerät (Fig.15) gespeichert wird.
So ist es z.B. möglich, der Diskette durch das Abspielgerät beim ersten Abspielen den Zeitpunkt des Abspielens mitzuteilen, z.B. durch einen mechanischen Code 53 (Fig.6), wie bei einer Lochkarte o.Ä. oder auf einen auf der Diskette befindlichen Magnetstreifen 50, (Fig.6), bzw. durch andere, ähnlich geeignete Maßnahmen.

Hierbei könnte der Abspielzeitpunkt durch eine geräteinterne Uhr, oder aber auch durch ein zusammen mit dem Duftspielfilm übertragenes Sendesignal übermittelt werden.

Nach diesem Zeitpunkt bleibt die Diskette dann noch für einen definierten Zeitpunkt ,z.B. für eine Woche abspielbar. Ist dieser Zeitpunkt überschritten, wird ein weiteres Abspielen vom Gerät verweigert. Auf diese Weise könnte trotz mehrmaligem Abspielen einer Diskette gewährleistet werden, daß die hierbei verwendeten Düfte sich beim Abspielen des jeweiligen Films mit Sicherheit noch in einem einwandfreien Zustand befinden.

Die Länge des möglichen Abspielzeitraumes, kann hierbei für alle Disketten einheitlich sein, oder auch an den jeweils empfindlichsten, der verwendeten Duftstoffe gekoppelt werden.

Eine derartige, zeitliche Abspielbegrenzung kann auch ohne vorheriges Abspielen der Diskette mit der Mindesthaltbarkeit der darauf verwendeten Düfte gekoppelt werden. So könnte eine z.B. im März 1997 verkaufte Diskette, deren eingelagerte Duftstoffe etwa bis zum Dezember 1999 perfekt haltbar sind, bei der Produktion gleichzeitig mit einem entsprechenden, z.B. mechanischen, optischen oder magnetischen Timecode versehen werden.

Bleibt diese Diskette dann sehr lange unbenutzt, und der Benutzer versucht, diese z.B. erst im Juni 1999 mit wohlmöglich nicht mehr ganz einwandfrei riechenden Düften abzuspielen, wird das Abspielen vom Abspielgerät entsprechend verweigert.

Desweiteren ist es auch möglich, daß das Abspielgerät, statt schlicht den Dienst zu verweigern, dem Benutzer alternativ oder zusätzlich einen Hinweis gibt, daß die Diskette schon zu alt ist.

Sofern es eines Tages möglich wird, Duftsensoren in ausreichender Qualität zu erschwinglichen Preisen zu fertigen, wäre es auch möglich, einen derartigen Duftqualitäts-Sensor direkt in das Abspielgerät einzubauen.
Eine Qualitätssicherung der Düfte könnte hierbei statt durch eine zeitliche Abspielbegrenzung der Duftdisketten, allein über die Qualitätskontrolle derartiger Sensoren verrichtet werden.

Da das individuelle Empfinden für Düfte eine wesentlich größere Spannweite hat, als bei anderen Sinnesreizen, wird es bei dem vorliegenden Ausführungsbeispiel in Fig.6 (neben den bereits oben beschriebenen Möglichkeiten der individuellen Duftbeeinflussung durch die allgemeine Einstellung der Duftintensität, Temperatur, etc. am Abspielgerät) möglich gemacht, eine duftbegleitete Film-Vorführung auch nach weiteren Besonderheiten der individuellen Vorstellung des Benutzers zu modifizieren, (Fig.15).

Zunächst werden hierbei die Düfte, bei denen eine gewisse Anzahl von Benutzern eine spezifische Abneigung haben könnten, in Duftklassen eingeteilt. Soweit ein Benutzer nun eine oder mehrere der vorhandenen Duftklassen aus einer bedufteten Film-, oder Musikvorführung, etc. ausschließen will, kann er das, indem er diese Duftklassen am Abspielgerät oder über Filtercodes 55, die an der Diskette selbst eingestellt werden können, abwählen. Die nicht gewünschten Düfte werden dann von dem Abspielgerät ignoriert und die entsprechenden Düfte werden übersprungen.

Gibt es beispielsweise Benutzer, die den Duft eines Parfums nur sehr eingeschränkt schätzen, stellen sie dies zunächst mit einem entsprechenden, an ihrem Abspielgerät anwählbaren Precode (z.B. "perf.not" o.ä.) oder an der Diskette über vorwählbare Raster bzw. Filtercodes 55 ein (Fig.6).
Wird nun ein Film gezeigt, in dem manchmal ein Parfum vorkommt, (z.B. "Pretty Woman", "Romeo und Julia" von F.Zeffirelli 1968, oder "Gone with the Wind"), wird die Vorführung eines Parfums bei einem entsprechend eingestellten Abspielgerät entweder ganz ausgelassen oder nur in stark abgeschwächter Form durchgeführt.

Für die zweite Form der abgeschwächten Düfte wird in das Gerät eine entsprechende Abschwächautomatik eingebaut und an die eingehenden Signale der quasi abgewählten Düfte gekoppelt.
Eine derartige Abschwächung von Düften kann z.B. eingerichtet werden, indem der Luftdurchfluß im Abspielgerät verringert wird oder durch eine zusätzliche Beimengung von Frischluft.

Entschließt sich schließlich ein anderer Benutzer mit anderen Präferenzen einen bedufteten Film zu sehen, stellt er am Abspielgerät entsprechend einen anderen, persönlichen Precode ein, so daß neben der bereits erwähnten, individuellen Regelbarkeit der allgemeinen Duftintensität bei Bedarf auch ein perfekt auf den einzelnen Benutzer abgestimmtes Duft-Filmerlebnis gezeigt werden kann.

Hierbei sind auch Precodes denkbar, die an andere Eigenschaften der Vorführung gekoppelt werden und die sich ebenfalls auf der Duft-CD 5 selbst befinden können.

So ist es beispielsweise möglich, die Duft-CD's selbst auf bestimmte Eigenarten festzulegen, z. B. länderspezifische Präferenzen der Duftwahrnehmung. So ist etwa bekannt, daß Europäer den Duft von Jasmin weitaus weniger schätzen, als dies in Asien der Fall ist und umgekehrt hat der Geruch einer Pizza in Europa einen höheren Stellenwert als beispielsweise in Japan.
Mit einem entsprechenden, z.B. länderspezifischen Precode 53 (Fig.6) der an der Duft-CD 5 selbst eingestellt werden kann, (z.B. über einen mechanischen Precode 53 oder über einen Magnetstreifen 50, Fig.6), ist es etwa möglich, zunächst eine weltweit einheitliche CD herzustellen und diese dann ohne Umstellung der Produktion an spezifische Erfordernisse (z.B. des Vertriebsgebietes) anzupassen.

Eine entsprechende (z.B. länderbezogene) Spezifizierung der abgespielten Duftvorführung kann auch über die mit dem Film mitgesendeten Signale erfolgen. So können Sendersignale, die ursprünglich mit einem Duft verbunden sind, der in dem entsprechenden Land nicht sonderlich geschätzt wird, über eine entsprechende Signalmodifikation im Sender entweder unterbunden oder länderspezifisch abgeschwächt werden.

Desweiteren kann es auch eingerichtet werden, die Intensität einer gesamten Duft-Vorführung oder andere, allgemeine Eigenschaften einer Vorführung (z.B. Wärmewerte oder Intervall-Profile), mit entsprechenden Precodes an der Duftdiskette selbst einzustellen, so wie dies weiter oben bereits als Einstellmöglichkeit für das Abspielgerät vorgeschlagen wurde.

In Kombination der verschiedenen Einstellmöglichkeiten- und Precodes ist es schließlich möglich, für jeden einzelnen Benutzer ein individuelles Benutzerprofil zu erstellen, welches am Abspielgerät gespeichert wird und vom Benutzer abgerufen werden kann.
Hierbei werden dann durch einen einzigen Knopfdruck o.ä., beispielsweise 5 verschiedene, benutzerorientierte Precodes, eine bevorzugte Intensitätsregelung, ein Wärme-, ein Intervall-, und ein Mengen-Profil gleichzeitig abgerufen und voreingestellt.

Ein derartiges Benutzerprofil kann sich auch nach weiteren Bedingungen richten, z.B. nach aktuellen Stimmungen oder nach sonstigen Eigenschaften des Benutzers, nach Jahreszeiten, etc.
Bestimmte Düfte sind z.B. dafür bekannt, Stimmungen positiv verändern zu können und so könnte die Vorführung ein und desselben Films etwa bei depressiver Stimmung anders ausfallen, als bei einer Hochstimmung des Benutzers.
Insgesamt ergeben sich hierbei mindestens vier Arten der Modifikation einer duftbegleiteten Film-, Musik-, oder sonstigen Vorführung:
Allgemein über eine Modifikation der Sendersignale oder an der Diskette und individuell über die Einstellung am Abspielgerät oder an der Diskette.

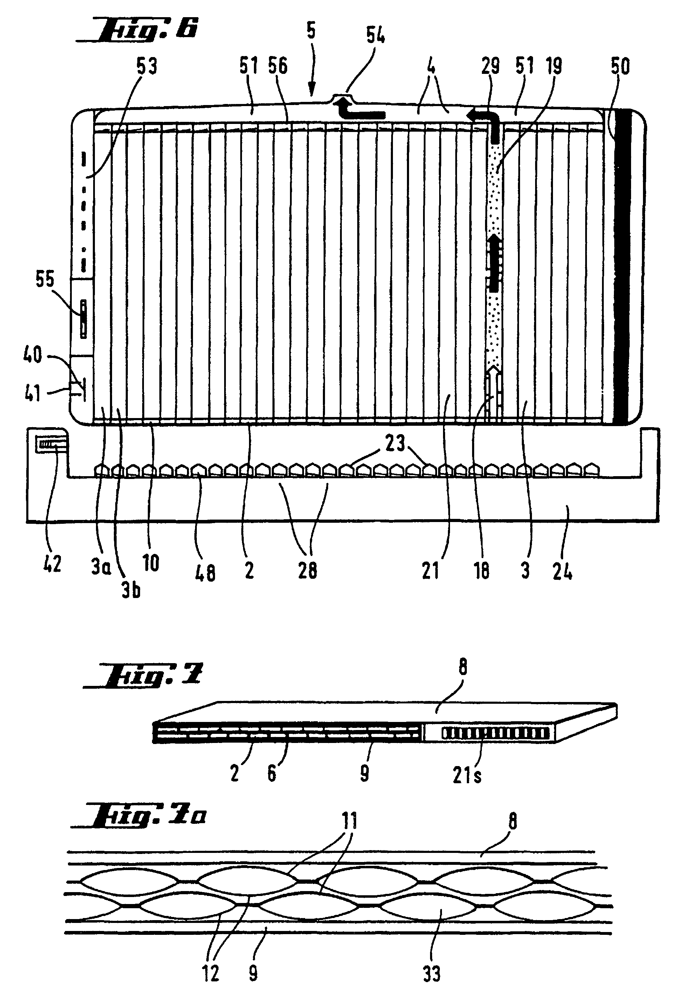

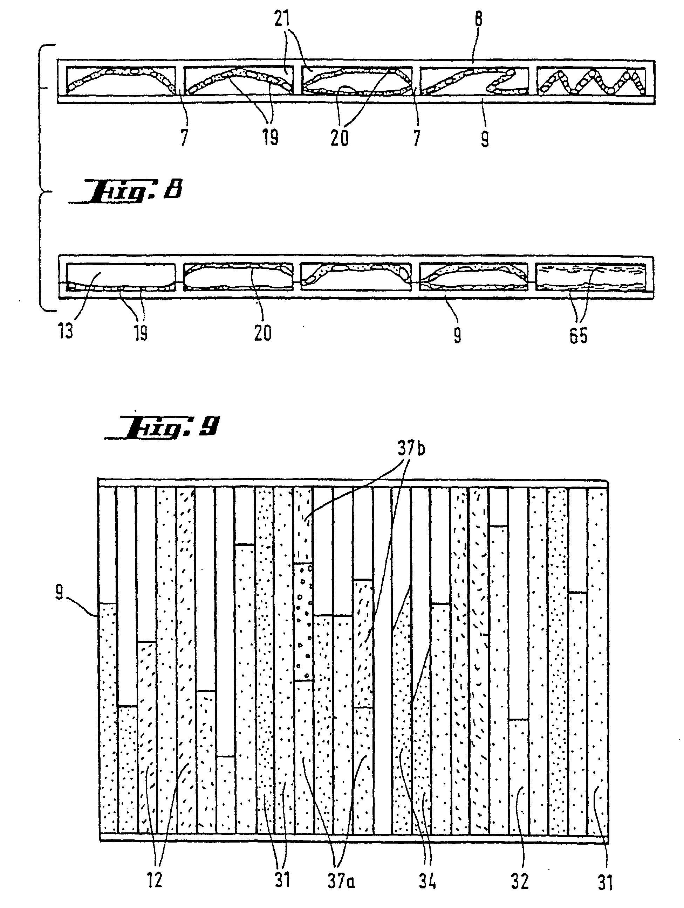

Bei dem in Fig.7 dargestellten Ausführungsbeispiel der Erfindung, werden die sehr dünnen Funktionsschichten in einer doppelten Duft-CD Anordnung untergebracht, sofern z.B. für das Abspielen eines besonders langen Films mehr Düfte benötigt werden, als auf einer einzelnen Scheibe untergebracht werden können. In diesem Fall dient die untere Diskettenhälfte 9 der oberen Duft-CD gleichzeitig als obere Diskettenhälfte 8 der unteren Duft-CD.

In einer besonders platzsparenden Abwandlung dieses Ausführungsbeispiels nach Fig.7a werden keine Zwischenschichten zwischen den beiden Duftschichten eingerichtet, sondern die einzelnen duftführenden Schichten so angeordnet, daß die Volumina der jeweils nächsten Schicht in den Lücken der davorliegenden Schicht zu liegen kommt.
Statt der zwei duftführenden Schichten ist es hierbei ebenfalls möglich, eine mittlere, duftführende Schicht zu verwenden, die von beiden Seiten mit Duftstreifen bedruckt wird, wobei auf beiden duftführenden Seiten der mittleren Schicht jeweils Trennfolien 12 angebracht werden.

Die Fläche, welche benötigt wird, um einen Duft insgesamt auf der Duft-CD unterzubringen, kann u.a. auch dadurch beeinflußt werden, wie intensiv die Duftmischung ist, die als Grundlage des Verkapselungs-Prozesses gewählt wird.

Sofern es sich um besonders intensive Mischungen handelt, die auf sehr kleinem Raum untergebracht werden können, ist es bei einer weiteren Abwandlung des Ausführungsbeispiels nach Fig.7 auch möglich, die in diesem Fall sehr schmalen duftführenden Schlitzkanäle 3s hochkant innerhalb von ebenfalls sehr schmalen Schutzröhren 21s unterzubringen.

Hierbei werden die schmalen Schutzröhren 21s nebeneinander hochkant stehend in der dabei immer noch sehr flachen Duft-CD angeordnet, etwa wie in Fig.7 im rechten Teil der Duft-CD dargestellt.

Bei dem in Fig.8 gezeigten und z.T. weiteren, nicht dargestellten Ausführungsbeispielen der Erfindung, werden die duftführenden Folien und Trennfolien 12 durch weitere Formen und Anordnungen ebenfalls so gestaltet, daß die dazwischen verkapselten Duftstoffe bei einem Durchfluß von Luft u.ä.m. von selbst aufbrechen und ihren zuvor noch verschlossenen und konservierten Duft freigeben.
So ist es beispielsweise möglich, die beiden, während des Herstellungsprozesses zusammen mit dem Duft aufeinandergeklebten Folien 11 und 12 mit verschiedenen Faltungen und Drehungen Fig.8 zu versehen, die durch die hindurchströmende Luft schließlich ent-faltet und ent-dreht werden, wobei die duftführende Folie 11 und die Trennfolie 12 ebenfalls voneinander gelöst werden und dabei ihren Duft selbsttätig freigeben.
Hierbei können während des Herstellungsprozesses auch kleine Duftstreifen oder -fasern spiralartig zusammengefasst oder -geklebt werden und zwar so, daß sich diese kleinen, duftführenden Spiralen bei der Vorführung des Duftes durch das in die Schutzröhren 21 einströmende Trägermedium, etwa Luft, entdrehen und den Duft dabei freigeben. Dies können in manchen Fällen auch nicht in Duftstoffkapseln gelagerte Düfte sein.

Bei einem weiteren, in Fig.9 dargestellten, bevorzugten Ausführungsbeispiel der Duft-CD wird das Drehen der S-CD für das Abspielen eines jeweils folgenden, neuen Duftes ebenfalls vermieden. Hierbei werden die einzelnen Duftbahnen 31 einfach nebeneinander angeordnet und später während des Abspielens über jeweils eigene Eingangsdüsen angespielt.

Soweit es nötig ist, während des Herstellungsprozesses einen weiteren, jedoch abgeschwächten Duft eines bereits vorhandenen, oder auch bei einer anderen Duft-CD bereits verwendeten Duftes auf eine Duftbahn 31 aufzutragen, ist es möglich, hierbei nur ein kurzes Stück der Duftbahn mit Duftspuren zu versehen, wobei sich eine modifizierte Duftbahn 32 ergibt. Hierdurch wird vermieden, daß dieser Duft noch einmal neu gemischt werden muß, was während des Herstellungsprozesses Aufwand ersparen kann.

Zur Vermeidung von neuen Mischarbeiten und zur Reduzierung der Menge an Düften, die vom Hersteller der Disketten insgesamt bevorratet werden müssen, ist es hier bei der Herstellung einer Duft-CD für einen neuen Film ebenso möglich, auf einer noch weiter modifizierten Multiduftbahn 36 mehrere, verschiedene Düfte zugleich aufzubringen, um daraus einen neu komponierten Mischduft zu erzeugen, sofern dieser neue Mischduft den Vorstellungen des Regisseurs des Films entspricht.
So ist es beispielsweise möglich, aus noch vorhandenen, frischen Duftbeständen zuvor hergestellter Duft-CD's anderer Filme, im ersten Teil 37a der neuen Muttiduftbahn 36 einen Blumenduft aufzutragen und im zweiten Teil 37b einen Wiesenduft, so daß dann daraus im Zusammenwirken beider Duftstoffe der Eindruck einer duftenden Blumenwiese entsteht.

Bei einer weiteren, modifizierten Duftbahn 34 dieses Ausführungsbeispiels werden die Duftstoffe so aufgebracht, daß sie sich bei normalem Luftdruck nur zum Teil entfalten. Erst bei einem verstärkten Luftdruck lösen sich hierbei die restlichen Teile der duftführenden Trennfolien 12 (wie z.B. in Fig.2b und 4b dargestellt). Entsprechendes kann auch durch verschiedene Verwinklungen der Folien erreicht werden.

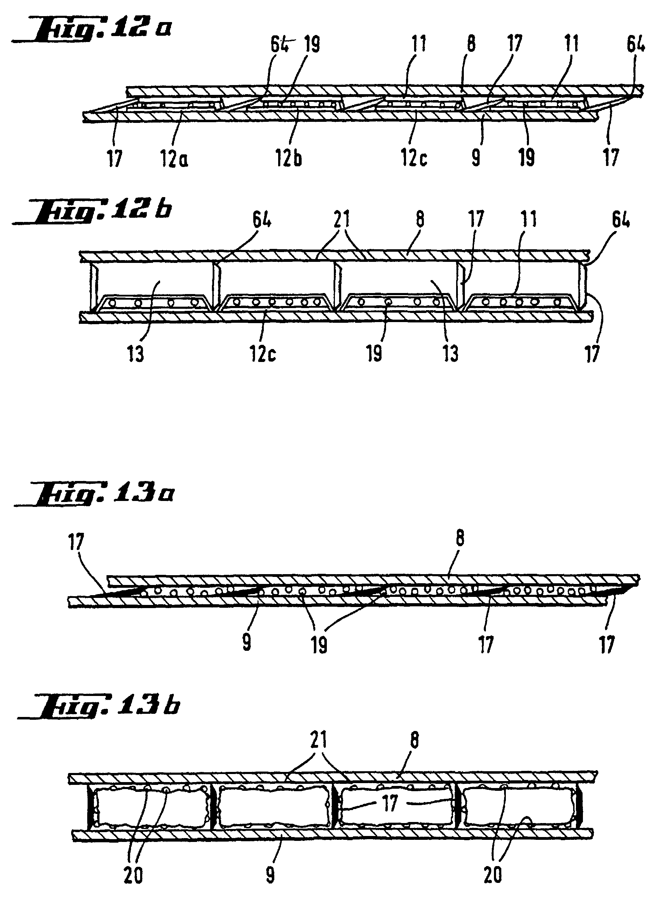

Bei einer weiteren, in Fig.10 und Fig.11 dargestellten Abwandlung der Duft-Kompaktdiskette 1 aus Fig.9 werden die in den Schlitzkanälen 3 aufgebrachten Mikroduft-Kapseln 19 nicht automatisch durch das mit dem Eindringen von Luft bedingte Ablösen der Trennfolien 12 aktiviert, sondern von Hand. Hierbei sind die Trennfolien im Bereich der Einlaßöffnungen 2 untereinander in einem griffstückartigen Außenteil, dem sogenannten Aktivator 61 verbunden.

Sofern der Anwender beabsichtigt, eine duftbegleitete Vorführung eines Spielfilms zu sehen, zieht er an dem Aktivator 61 der Trennfolien (Fig.11),
wobei diese alle zusammen von der anderen, duftführenden Folie abgezogen und hierdurch aktiviert werden. Anschließend wird diese aktivierte Diskette in das Abspielgerät (Fig. 15) eingesetzt, wobei die zugleich aktivierten Düfte sich dadurch, daß sie weiterhin in den Schutzröhren untergebracht bleiben, nicht vermischen oder nach außen treten.
Durch diese Abwandlung der Diskette ist es möglich, den notwendigen Luftdruck des Abspielgerätes deutlich herabzusetzen, da dieser nun nicht mehr so stark sein muß, daß sich hierdurch die Trennfolien 12 selbsttätig von der Folienoberseite 11 ablösen, wobei die Düfte hier ebenfalls bis kurz vor dem Anwendungsfall in den Duftkapseln 19 aktiviert bleiben.
Bei einer weiteren, nicht dargestellten Abwandlung dieses Ausführungsbeispiels, werden beispielsweise auf der duftführenden Folienoberseite 11 neuartige, duftführende Aufträge, sogenannte duftführende Touchcoatings 58 verwendet. Die duftführenden Touchcoatings 58 werden nun nicht dadurch aktiviert, daß zwei Folien 11 und 12 voneinander getrennt werden, sondern bereits allein dadurch, daß die Oberfläche des Touchcoatings 58 berührt wird, oder dadurch, daß ein Gegenstand darüber streicht.
In diesem Fall werden die Touchcoatings 58 vorzugsweise innerhalb der Duft-CD z.B. direkt auf eine der beiden Diskettenhälften 8 oder 9 einseitig aufgetragen, oder auf eine der duftführenden Folien 11 oder 12. Vor den duftführenden Teilen 11/12/8/9 wird nun ein Aktivator 61 installiert, welcher vor dem Abspielen manuell oder automatisch durch das Abspielgerät entfernt wird. Die duftführenden Teile 11/12/8/9 und der Aktivator 61 bilden hierbei zusammen die Duftmatrix 60
Durch das Entfernen des Aktivators 61 werden die in der Duftmatrix 60 befindlichen Touchcoatings 58 nun so berührt, daß hierbei die in dem Coating befindlichen Duftstoffe aktiviert werden. Gleich anschließend werden die nun in der Duft-CD aktivierten Düfte, im wesentlichen ähnlich, wie in den bereits beschriebenen Ausführungsbeispielen zuvor, über bestimmte Signale aufgerufen und vorgeführt.

Bei anderen Variationen der Erfindung, werden duftführende Coatings mit mikroverkapselten Duftstoffen versehen, die durch Vakuum, Licht, Wärme, oder andere Auslöser aktiviert werden können.
Bevor die Düfte einer derartigen Diskette abgespielt werden können, werden die inhärenten Duftstoffe nun zuerst durch einen entsprechenden Auslöser aktiviert und für den Abspielvorgang vorbereitet.

So können etwa lichtempfindliche Duftstoffe, bzw. lichtempfindliche Versiegelungen aktiviert werden, indem vor dem Abspielvorgang eine Lichtquelle, z.B. ein Laserstrahl in die Duftbahnen gelenkt wird.
Soweit derartige Duftbahnen zusätzlich durch Ein- und Auslaß-Versiegelungen 10 und 22 verschlossen sind, werden diese Siegel vor dem Eindringen des Auslösers, z.B. des Laserstrahls, entfernt oder unwirksam gemacht.

Bei einer weiteren Variation dieses Ausführungsbeispiels werden die Disketten so geformt, wie bereits in der Fig.1 dargestellt, als runder Flachspeicher. In diesem Fall sind die beiden Diskettenhälften 8 und 9 im wesentlichen nur teilweise miteinander verbunden. Die Verbindung der Diskettenhälften besteht hierbei vorzugsweise so, daß die Diskettenhälften zwar nicht voneinander getrennt, aber um die Mittelachse herum gegeneinander verdreht werden können.
Soweit eine derartige Diskette aktiviert werden soll, werden nun die obere und die untere Diskettenhälfte 8 und 9 um die Achse herum, ein kurzes Stück gegeneinander verdreht, wobei die darinliegenden, duftführenden Touchcoatings 58 durch das Darüberstreichen eines inhärenten oder externen Auslösers aktiviert werden und die Diskette abspielbereit wird.

Bei einer weiteren, in Fig.12a und b. dargestellten Ausführungsform der Erfindung wird die Duft-CD für den Transport, Versand und die Aufbewahrung noch flacher, als bei den bisherigen Ausführungsformen hergestellt, indem hier auf eine Durchflußmöglichkeit von Luft, etc. gänzlich verzichtet wird. Der in Fig.2a und b, sowie 4a und b gezeigte Verdrängungs-Hohlraum 13, oder sonstige Hohlräume, die einen Luftdurchfluß an den inhärenten Duftstoffen vorbei ermöglichen, sind bei diesem Ausführungsbeispiel während des Transportes (Fig. 12a) somit noch nicht als Ausdehnung vorhanden, so daß eine derartige Duft-CD noch näher an die Durchmesser-Maße einer Musik-CD / CD-ROM heranreicht.

Soweit diese Ausführungsform der Duft-CD nun abgespielt werden soll, wird diese vor dem eigentlichen Abspielvorgang zunächst durch den Benutzer, oder automatisch durch das Abspielgerät auf ein etwas größeres Volumen, als ihr Transportvolumen vergrößert.
Diese Volumenvergrößerung kann beispielsweise geschehen, indem die obere und die untere Diskettenhälfte 8 und 9 von außen mit einem kleinen, seitlichen Eingriff 35 versehen wird, wobei der Benutzer z.B. ein Geldstück in diesen seitlichen Eingriff 35 einsetzt und dreht, wodurch die Duft-CD-Hälften 8 und 9 ein kleines Stück voneinander abrücken. Durch einen kleinen Abstandhalter 38 bleibt dieser Abstand zwischen den CD-Hälften nun während des Abspielvorganges erhalten, wobei das Aufklappen der Duft-CD auch automatisch durch das Abspielgerät erfolgen kann.
Erst nach diesem Aufklappvorgang wird hierbei der notwendige Platz für das Vorbeistreichen von Luft oder anderer Medien an den in den Schlitzkanälen 3 der Duft-CD gelagerten Duftstoffen, oder an anderen, inhärenten Duftkontaktflächen eingerichtet (Fig. 12b).
Um hierbei zu gewährleisten, daß sich um die Schlitzkanäle 3 oder andere duftführende Flächen herum weiterhin luftdichte Röhren zur eindeutigen Ausrichtung des Luftstroms bilden, werden bei diesem Ausführungsbeispiel die bisherigen Trennstege durch modifizierte flexible Trennstege 17 ersetzt, welche sich während der Volumenvergrößerung aufrichten, ohne dabei abzubrechen.
Hierbei können die Enden der flexiblen Trennstege 17 beispielsweise über Kunststoff-Scharniere 64 mit den Diskettenhälften 8 und 9 verbunden sein.

Bei einer weiteren, in Fig.13a und 13b dargestellten Abwandlung des Ausführungsbeispiels nach Fig. 12a und b, werden gleichzeitig mit dem in Fig.12a und b beschriebenen Vorgang des Aufklappens der oberen und unteren Diskettenhälften 8 und 9 gleichzeitig darin befindliche Mikroduft-Kapseln 19 aktiviert, indem die duftführenden Folien 8 und 9 bereits durch den Vorgang des Aufklappens zumindest im mittleren Bereich voneinander getrennt werden.
Die heranströmende Luft o.ä. muß hierbei nun nicht mehr die Kraft aufbringen, um die duftführenden Folien 8 und 9 voneinander zu trennen, sondern kann ungehindert an den bereits aufgeplatzten Duftkapseln 19 vorbeifließen.
In diesem Fall können die Mikroduft-Kapseln 19 auch bevorzugt so aufgebracht werden, daß sich in der Schutzröhre 21 kein, bzw. kein vollständiger Schlitzkanal 3, bzw. duftführende Folien 11 und/oder 12 mehr befindet, (Fig.13a).

Hierbei werden die Mikroduft-Kapseln 19 schließlich direkt auf die obere 8 und/oder die untere Diskettenhälfte 9 aufgetragen bzw. eingeklebt, wobei die Diskettenhälften 8 und 9 schließlich über flexible Trennstege 17 verbunden und so zusammengeklappt werden, daß die Duftkapseln 19 weitgehend direkt zwischen die Diskettenhälften 8 und 9 zu liegen kommen (Fig. 13a).
Beim Aufklappen der Diskettenhälften 8 und 9 richten sich nun die flach dazwischen liegenden, flexiblen Trennstege 17 auf, wobei einerseits die dazwischen eingeklebten Mikroduft-Kapseln 19 auseinandergerissen werden und zu den aufgeplatzten Kapseln 20 werden, während andererseits mit der beim Aufklappen entstehenden Volumenvergrößerung gleichzeitig das für den Durchfluß von Luft o.ä. nötige Volumen in der Diskette eingerichtet wird (Fig.13b.).

Bei einer weiteren, nicht dargestellten Variation dieser Ausführungsform einer Diskette ist ein Verdrängungs-Hohlraum 13, oder sonstige Hohlräume, die einen Luftdurchfluß an den darinliegenden Duftstoffen vorbei ermöglichen, im Transport- oder Verschlußzustand ebenfalls noch nicht als Ausdehnung vorhanden. Diese Diskettenvariation besitzt gleichzeitig auch keine mikroverkapselten Duftstoffe, sondern hier werden die Duftstoffe mit anderen, weiter oben beschriebenen Verfahren auf die Diskette aufgetragen, um durch die Weglassung des u.U. zeitaufwendigen Verkapselungsverfahrens eine schnelle und sehr kurzfristige Herstellung von Disketten für ein bestimmtes Ereignis zu ermöglichen.
Die wesentlich schlechteren Konservierungsmöglicheiten für derartige Disketten mit nicht verkapselten Duftstoffen werden bei dieser Variation einer Duftdiskette nun teilweise dadurch ausgeglichen, indem der im Verschlußzustand noch fehlende Hohlraum 13 über den Duftstoffbahnen so dicht angelegt wird, daß hierdurch ein Konservierungseffekt eintritt (ähnlich, wie in den Ausführungsbeispielen in Fig. 12a und b oder 13a /b).
Hierbei wird durch den weitgehenden Ausschluß jeder Art von Hohlraum oberhalb der Duftstoffbahnen eine Art Versiegelung der Duftstoffe erreicht, so daß das Eindringen von Sauerstoff und anderen, den Alterungsprozess beschleunigenden Stoffen, verhindert wird.

Soweit diese, für den kurzfristigen Verbrauch bestimmten Disketten nun kurz vor dem Abspielen aufgeklappt werden, bilden sich hier ebenfalls die Hohlräume 13, welche für den Durchfluß von ausreichenden Luftmengen erforderlich sind. (etwa so, wie in der Veränderung des vorigen Ausführungsbeispiels von Fig. 13a nach Fig. 13b).

Bei einer weiteren, nicht dargestellten Abwandlung des Ausführungsbeispiels, werden ebenfalls keine oder nur sehr geringe, innere Volumina für den Durchfluß von Luft, etc. eingerichtet. Hierbei werden aber zunächst zumindest die Schlitzkanäle 3 ganz oder teilweise elastisch ausgeführt, so daß sich bei der Aktivierung eines duftführenden Schlitzkanals 3 zunächst die Wände des Schlitzkanals so weit ausdehnen, wie noch Volumina um den Kanal herum vorhanden sind, wobei die herangepumpte Luft nun hindurch, an dufttragenden Materialien 26, an Mikroduft-Kapseln 19, oder anderen, darin befindlichen Duftspeichern vorbei fließen kann.
Da sich der hierbei in einem aktivierten Schlitzkanal 3 entstehende Druck auch auf die Schutzschichten (z.B. Schutzröhren 21) um den Schlitzkanal herum überträgt, werden diese so gestaltet, daß sie während der Aktivierung ebenfalls kurzzeitig nachgeben können und so den Luftdurchfluß in der vorgesehenen Größenordnung ermöglichen.
Ein derartiger, elastischer Schlitzkanal kann ebenfalls in anderen, der beschriebenen Ausführungsbeispiele verwendet werden.

Mit den zuletzt beschriebenen Ausführungsbeispielen ist es schließlich möglich, die Duftstoffe, die selbst fast kein Volumen benötigen, für den Transport extrem kleinflächig zu halten, während bei deren Vorführung gleichzeitig die Querschnitte von an dem Duftstoff vorbeifließenden Luftvolumina groß gehalten werden können.

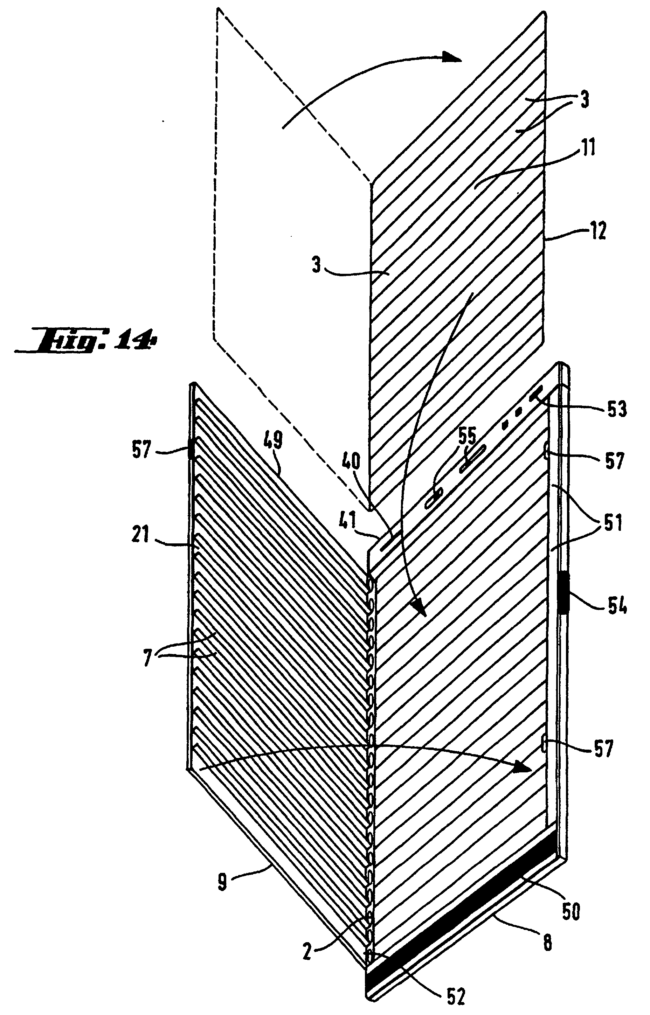

Bei einem weiteren, in Fig.14 dargestellten Ausführungsbeispiel der Erfindung, handelt es sich um eine Variation des Ausführungsbeispiels nach Fig.6.
Hierbei wird aus Gründen eines einfacheren Recyclings der abgespielten Disketten und ebenfalls zur Vereinfachung der Herstellung zunächst ein Kunststoff-Scharnier 52 zwischen die Enden der oberen 8 und unteren Diskettenhälfte 9 gefügt, (Fig.14).

Bei der Herstellung einer derartigen, einstückigen Klappdiskette 49 müssen die Diskettenhälften 8 und 9 nicht mehr passgenau zugeführt werden, sondern sind durch das Kunststoff-Scharnier 52 bereits fest miteinander verbunden und zueinander fixiert.
Die duftführenden Folien 11 und 12 müssen nach ihrer Herstellung und Zusammenfügung nun nur noch zwischen die Diskettenhälften 8 und 9 der Klappdiskette 49 gesteckt werden, worauf die Diskettenhälften zusammengeklappt werden und die duftführenden Folienschichten 11 und 12 in sich fixieren.

Um ebenfalls eine u.U. schwer lösbare Verschweißung der Diskettenhälften 8 und 9 am anderen Ende der Klappdiskette 49 zu vermeiden, werden dort ein oder mehrere Einrastvorsprünge 57 eingerichtet, welche beim Zusammenklappen der Diskettenhälften 8 und 9 einrasten und die Klappdiskette somit im zusammengeklappten Zustand fest fixieren. Die Einrastvorsprünge 57 werden dabei vorzugsweise so an der Klappdiskette 49 angebracht, daß sie nicht vom Benutzer, sondern nur mit speziellen Geräten geöffnet werden können.
Da die äußere Dichtigkeit einer Klappdiskette 49 oder deren Schutzröhren 21 u.U. nicht so weit reicht, wie bei einer zusammengeschweißten Duft-CD, werden die inneren, duftführenden Folien 11 und 12 hier vorzugsweise so ausgeführt, daß sie als duftführender Flachkanal 33 erhalten bleiben,
wobei die durchfließenden Luftmassen allein durch den Flachkanal 33 weitergeführt werden und hierbei keiner
weiteren Abdichtung durch die Schutzröhren 21 bedürfen.

Soweit die Diskette schließlich abgespielt ist und im Zuge eines Recyclings die inneren, duftführenden Schichten 11 u. 12 und Kanäle 33 von den Diskettenhälften getrennt werden sollen, wird die Klappdiskette 49 nun durch eine spezielle Vorrichtung geöffnet, die Schichten 11 und 12 oder 33 o.ä. werden entfernt und die Diskette wird anschließend mit neuen, duftführenden Folien bestückt. Soweit bei der Entsorgung auch die Schutzhüllen, bzw. die Diskettenhälften 8 und 9 entsorgt werden sollen, können diese nach der Trennung von den duftführenden Folien ebenfalls dem Recycling zugeführt werden. Entsprechend können für die Diskettenhälften 8 und 9 bzw. für die Klappdiskette 49 Materialien verwendet werden, welche sowohl den Funktionszweck erfüllen, als auch biologisch abbaubar und/oder wiederverwertbar sind.

Bei einer weiteren Variation des Ausführungsbeispiels der Erfindung nach Fig. 14 werden die Verbindungen zwischen der oberen 8 und der unteren 9 Diskettenhälfte, trotz der Klappform zugleich als feste Kleb- Schweiß-, etc. Verbindung ausgeführt. Diese Verbindungen werden dabei vorzugsweise so ausgeführt, daß sie zum Zwecke der Entsorgung einer Diskette aufbrechen können, um die duftführenden Schichten zu entfernen.

Bei einer weiteren, nicht dargestellten Ausführungsform der Erfindung, werden die einzelnen duftführenden Folien hintereinander statt nebeneinander zusammengefügt, so daß sich hierdurch insgesamt ein duftführendes Band ergibt, auf welchem sich alle Düfte einer beabsichtigten Vorführung befinden. Das duftführende Band besteht dabei ebenfalls aus einer oberen und einer unteren Folie zwischen denen die Duftstoffe vorzugsweise in Form von Mikrokapseln gelagert sind.
Die Länge der duftführenden Streifen auf diesem Band ist dabei normiert, so daß ein neuer Duft durch das Vorspulen des Zweifolien-Bandes um eine definierte Länge genau lokalisiert werden kann.

Soweit nun das Abspielen eines Duftes beabsichtigt ist, spult das Zweifolien-Band durch entsprechende Signale gesteuert, genau so weit vor, daß der Streifen mit dem vorgesehenen Duft vor einem Schutzkanal zu liegen kommt.
Hierbei werden die duftführenden Folien des Bandes voneinander getrennt, so daß diese nun im Bereich des Schutzkanals frei liegen und die Duftkapseln geöffnet werden.
Der Schutzkanal führt die von den geöffneten Mikrokapseln abgegebene, beduftete Luft nun zu einem Ausgang. Hierbei ist ein Schutzkanal für den Transport mehrerer, bzw. für den jeweils abzuspielenden Duft vorgesehen.

Diese Aufbewahrungsform in der Art eines Duftbandes, ist insbesondere geeignet, sofern das wiederholte Abspielen ein und desselben Duftes oder nur weniger verschiedener Düfte vorgesehen ist.
In dieser Form handelt es sich somit vorzugsweise um eine Konservierungsart für die Vorführung eines einzigen oder nur weniger Düfte, die über einen längeren Zeitraum hinweg, jeweils in einwandfreier Form vorgeführt werden sollen.

Bei weiteren, nicht dargestellten Anwendungen der Erfindung wird die Duft-CD z.B. in Kombination mit einem Musik-CD Player oder während des Ablaufs eines Computerspiels abgespielt.
Andere Anwendungen ergeben sich, sofern die Duft-CD von einem Nahrungsmittel- oder Getränkeautomat für vorbeigehende, interessierte Kunden auf Knopfdruck oder automatisch abgespielt wird. Hierbei ist es möglich, daß auf der Duft-CD nicht verschiedene, sondern z.B. 50 mal derselbe Duft aufgebracht wird, wobei die Duft-CD hier hauptsächlich statt eines flüssigen großvolumigen Duftbehälters verwendet wird, um eine optimale Frischhaltung der dargebotenen Düfte zu gewährleisten, was bei flüssigen und ständig mit Luft kontaktierten Duftvorräten meist nicht dauerhaft möglich ist (siehe der vorvorige Absatz).

Schließlich können derartige Duft-CD's, die es erstmals möglich machen, Düfte und auch Sets von sehr vielen verschiedenen Düften an jedem gewünschten Ort, zu bestimmten Ereignissen automatisch in perfekter Qualität abzuspielen und dies bei Bedarf auch individuell auf das persönliche Empfinden des Konsumenten oder Betrachters abgestimmt, für die verschiedensten weiteren Anwendungen verwendet werden.
Dies insbesondere auch dann, soweit bei diesen Anwendungen bisher auf die Verwendung von Dufteindrücken verzichtet werden mußte, da, wenn überhaupt, Düfte nur allgemein in einen Raum geblasen werden konnten.
Hierdurch könnten sich viele Konsumenten, denen gerade nicht nach diesem Duft zumute ist, oder denen dieser Dufteindruck zu stark oder zu schwach erscheint, gestört fühlen.
Ein dezentraler Multiduftspeicher, wie in der vorliegenden Erfindung vorgeschlagen, der Düfte in der gewünschten Art und Qualität abspielt, kann dieses Problem des Alle-über-einen-Kamm-Scheren vermeiden und für jeden Konsumenten ein angenehmes Dufterlebnis an dem Ort, zu der Zeit und in der Art und Stärke ermöglichen, die er sich persönlich wünscht.

Bei weiteren, nicht näher ausgeführten Ausführungsbeispielen der Erfindung werden verschiedenartige Kombinationen aus den beschriebenen Ausführungsbeispielen oder Teilen hiervon zusammengesetzt.

### Bezugszeichenliste

- 1.): Duft-Kompaktdiskette / Duft-CD
- 2.): Einlaßöffnung
- 3.): Schlitzkanal
- 3a.): Schlitzkanal Duft A
- 3b.): Schlitzkanal Duft B
- 3c.): Schlitzkanal Duft C
- 3d.): Schlitzkanal Duft D
- 3k.): modifizierterSchlitzkanal
- 3s.): schmaler Schlitzkanal
- 4.): Auslaß-Panel
- 5.): Duft-CD
- 6.): Kanal-Trennung
- 7.): Trennstege
- 8.): obere Diskettenhälfte
- 9.): untere Diskettenhälfte
- 10.): Einlaß-Versiegelung
- 11.): dufführende Folienoberseite
- 12.): duftführende Trennfolien
- 12a.): Trennfolie Duft A
- 12b.): Trennfolie Duft B
- 12c.): Trennfolie Duft C
- 12d.): Trennfolie Duft D
- 13.): Verdrängungs-Hohlraum
- 14.): Ausgang
- 15.): duftführender Folienteil
- 16.): duftfreier Folienteil
- 17.): flexible Trennstege
- 18.): duftstoffreie Luft
- 19.): Mikroduft-Kapseln
- 20.): aufgeplatzte Duftkapseln
- 21.): Schutzröhre
- 21a.): Schutzröhre Duft A
- 21b.): Schutzröhre Duft B
- 21s.): schmale Schutzröhre
- 22.): Auslaßversiegelung
- 23.): Eingangsdüse
- 24.): Abspielgerät
- 25.): Schlitzkanal-Auslaß
- 26.): dufttragende Materialien
- 27.): Ausgangs-Düse
- 28.): Eingangsdüsen-Panel
- 29.): duftstoffangereicherte Luft
- 30.): Folienverbindung
- 31.): Duftbahnen
- 32.): modifizierte Duftbahn
- 33.): duftführender Flachkanal
- 34.): modifizierte Duftbahn
- 35.): seitlicher Eingriff
- 36.): modifizierte Multiduftbahn
- 37a.): Teil- Duftbahn
- 37b.): Teil- Duftbahn
- 38.): Abstandhalter
- 39.): Schweißverbindung
- 40.): Markierzunge
- 41.): Abtastöffnung
- 42.): Abtaststift
- 43.): Steck- od. Klebverbindung
- 44.): modifzierter Flachkanal
- 45.): Verbindung
- 46.): obere Trennstege
- 47.): Verbindung
- 48.): Ventil
- 49.): Klappdiskette
- 50.): Magnetstreifen
- 51.): Luftstrom-Ausrichter
- 52.): Kunststoff-Scharnier
- 53.): Ländercode
- 54.): Fokus-Ausgangsdüse
- 55.): individueller Filtercode
- 56.): Auslaß-Ventil
- 57.): Einrastvorsprünge
- 58.): duftführendes Touchcoating
- 59.): Aktivator-Folie
- 60.): Duftmatrix
- 61.): Aktivator
- 62.): Oxydationshemmer
- 63.): Vakuum
- 64.): Kunststoff-Scharnier
- 65.): Vlies
- 66.): Membranpumpe
- 67.): Signalempfänger
- 68.): Wärmeregler
- 69.): Mengenregler
- 70.): Magnetstreifen-Leser

## Patentansprüche

1. Vorrichtung zur Bereitstellung von in die Luft oder in Luftgemische abzugebenden Stoffen, insbesondere von Duftstoffen, mit einem flachen, scheiben- oder plattenförmigen Grundkörper (8,9), der im wesentlichen parallel zu seiner Ober- und/oder Unterseite von mehreren separaten Kanälen (3) durchsetzt ist, die zur Aufnahme der abzugebenden Stoffe dienen und je eine Ein- und Auslaßöffnung (25) enthalten und von einem der Einlaßöffnung zugeführten Gasstrom durchströmbar sind, wobei die Ein- und Auslaßöffnung zumindest eines Kanals (3) bis zur Abgabe des Stoffes gasdicht verschlossen und/oder der Stoff in zumindest einen Kanal (3) in einem Speicher gasdicht eingebracht ist, der erst zum Abgabezeitpunkt des Stoffes diesen freisetzt.

2. Vorrichtung nach Anspruch 1, **dadurch gekennzeichnet, daß** zumindest eine Ein- und Auslaßöffnung mit einem zerstörbaren Siegel verschlossen ist.

3. Vorrichtung nach Anspruch 1, **dadurch gekennzeichnet, daß** der verschlossene Kanal im Inneren zumindest den abzugebenden Stoff und/oder ein Inertgas enthält.

4. Vorrichtung nach einem der vorstehenden Ansprüche, **dadurch gekennzeichnet, daß** der Speicher den Stoff in einem Trägermaterial so eingeschlossen enthält, daß der Stoff durch Zerstörung des Trägermaterials freisetzbar ist.

5. Vorrichtung nach einem der vorstehenden Ansprüche, **gekennzeichnet durch** Betätigungsmittel, die zur Aktivierung der Stoffabgabe an die Umgebungsluft oder an dem der Einlaßöffnung zugeführten Gasstrom eine Öffnung oder Zerstörung der gasdichten Verschlüsse der Eintritts- und/oder Austrittsöffnung bewirken und/oder das Trägermaterial für die Stoffe zerstören.

6. Vorrichtung nach einem der vorstehenden Ansprüche, **gekennzeichnet durch** Mittel zur gezielten Zerstörung des gasdichten Verschlusses oder des Trägermaterials im Hohlkörper oder Kanal.

7. Vorrichtung nach Anspruch 6, **dadurch gekennzeichnet, daß** die Mittel zur gezielten Zerstörung in der Vorrichtung selbst angebracht sind.

8. Vorrichtung nach Anspruch 6, **dadurch gekennzeichnet, daß** die Mittel zur Zerstörung getrennt von der Vorrichtung derart vorgesehen sind, daß sie bei Aktivierung der Abgabe der Stoffe mit der Vorrichtung in Wirkverbindung bringbar sind, wobei die Mittel vorzugsweise so ausgebildet sind, daß sie beim Einlegen der Vorrichtung in ein Gerät zur Abgabe der gasförmigen Stoffe wirksam werden.

9. Vorrichtung, insbesondere nach einem der vorstehenden Ansprüche, zur Bereitstellung von gasförmig abzugebenden Stoffen, insbesondere von Duftstoffen,
**gekennzeichnet durch**
- zumindest einen mit wenigstens einer Gasaustauschöffnung (25) versehenen Hohlkörper (8, 9), dessen Inneres (3) von einer Gasströmung durchspülbar ist;
- zumindest einen in dem Hohlkörper (8, 9) angeordneten Speicher (19) für den in eine Umgebung abzugebenden Stoff, der von einem Trägermaterial (10, 22; 11) so eingeschlossen ist, daß der Stoff **durch** Zerstörung zumindest eines Teils des Trägermaterials (10, 22; 11) freisetzbar ist;
- Mittel (23) zur gezielten Zerstörung des Trägermaterials (10, 22; 11) im Hohlkörper, wobei die Mittel zur gezielten Zerstörung in der Vorrichtung selbst angebracht sind oder die Mittel zur Zerstörung getrennt von der Vorrichtung derart vorgesehen sind, daß sie bei Aktivierung der Abgabe der Stoffe mit der Vorrichtung in Wirkverbindung bringbar sind, wobei die Mittel vorzugsweise so ausgebildet sind, daß sie beim Einlegen der Vorrichtung in ein Gerät zur Abgabe der gasförmigen Stoffe wirksam werden.

10. Vorrichtung nach Anspruch 9, **dadurch gekennzeichnet, daß** die Hohlkörper als Kanäle ausgebildet sind.

11. Vorrichtung nach einem der Ansprüche 9 oder 10, **dadurch gekennzeichnet, daß** die Mittel zur gezielten Zerstörung der gasdichten Verschlüsse und/oder des Trägermaterials mechanische und/oder pneumatische Mittel sind.

12. Vorrichtung nach einem der Ansprüche 4 bis 11, **dadurch gekennzeichnet, daß** der Hohlkörper oder die Kanäle gegeneinander verschiebbare Wandungselemente aufweisen, mit denen das Trägermaterial in Wirkverbindung steht.

13. Vorrichtung nach einem der Ansprüche 4 bis 12, **dadurch gekennzeichnet, daß** das Trägermaterial benachbart zur Gasaustauschöffnung des Hohlkörpers oder der Eintrittsöffnung des Kanals derart angeordnet ist, daß ein durch die Gasaustauschöffnung oder Gasteingangsöffnung in das Innere des Hohlkörpers oder Kanals gerichteter Gasstrahl zumindest einen Teil des im Hohlkörper oder Kanal angeordneten Trägermaterials zerstört.

14. Vorrichtung nach einem der Ansprüche 4 bis 13, **dadurch gekennzeichnet, daß** das Trägermaterial so im Hohlkörper oder in einem der Kanäle angeordnet ist, daß es dessen Querschnitt im wesentlichen verschließt, so daß beim erstmaligen Einblasen von Gas in die Gaseintrittsöffnung des Hohlkörpers das Trägermaterial zur Freisetzung des Stoffes aufgebrochen wird.

15. Vorrichtung nach einem der vorstehenden Ansprüche, **dadurch gekennzeichnet, daß** in dem Hohlkörper oder Kanal ein mit dem abzugebenden Stoff angereichertes oder getränktes Absorptionsmedium angeordnet ist.

16. Vorrichtung nach einem der vorstehenden Ansprüche, **dadurch gekennzeichnet, daß** der Speicher vom Trägermaterial zumindest teilweise eingeschlossene Mikrokapseln enthält, in die der freizusetzende Stoff eingeschlossen ist.

17. Vorrichtung nach einem der vorstehenden Ansprüche, **dadurch gekennzeichnet, daß** eine Mehrzahl von Hohlkörpern oder der Grundkörper aus einer oberseitigen Abdeckschicht (8), einer unteren Abdeckschicht (9) sowie die Hohlkörper oder Kanäle voneinander abgrenzende Trennstege (7) aufgebaut sind.

18. Vorrichtung nach einem der Ansprüche 4 bis 17, **dadurch gekennzeichnet, daß** das Trägermaterial eine Hüllschicht (11), eine weitere, mit der Hüllschicht (11) lösbar verbundene Hüllschicht (12), sowie ein die beiden Hüllschichten (11, 12) verbindendes Haftmittel enthält, in welche der Speicher eingebettet ist.

19. Vorrichtung nach einem der vorhergehenden Ansprüche, **dadurch gekennzeichnet, daß** zumindest eine Gasaustauschöffnung des Hohlkörpers versiegelbar ist.

20. Vorrichtung nach einem der vorstehenden Ansprüche 2 bis 9, **dadurch gekennzeichnet, daß** eine Mehrzahl von Hohlkörpern oder Kanälen nebeneinander angeordnet ist.

21. Vorrichtung nach Anspruch 20, **dadurch gekennzeichnet, daß** die Hohlkörper oder Kanäle bezogen auf ein Zentrum im wesentlichen radial verlaufend angeordnet sind.

22. Vorrichtung nach Anspruch 20, **dadurch gekennzeichnet, daß** die Hohlkörper oder Kanäle zumindest teilweise schräg oder parallel zueinander verlaufend angeordnet sind.

23. Vorrichtung nach einem der vorstehenden Ansprüche, **dadurch gekennzeichnet, daß** zumindest die Mehrzahl der Hohlkörper oder Kanäle nebeneinander und/oder übereinander angeordnet ist.

24. Vorrichtung nach einem der vorhergehenden Ansprüche, **dadurch gekennzeichnet, daß** der Speicher nur einen Teil des Hohlkörpers einnimmt.

25. Vorrichtung nach einem der vorhergehenden Ansprüche, **dadurch gekennzeichnet, daß** diese zumindest teilweise aus biologisch abbaubarem Material aufgebaut ist.

26. Vorrichtung nach einem der vorstehenden Ansprüche **dadurch gekennzeichnet, daß** der Speicher zumindest benachbart zu einer der Öffnungen des Hohlkörpers derart in dem Hohlkörper oder Kanal befestigt ist, daß er den Querschnitt des Hohlkörpers oder Kanals im wesentlichen verschließt, sowie ein
Mittel aufweist, um Gas zumindest einer Öffnung des Hohlkörpers steuerbar zuzuführen.

27. Vorrichtung nach einem der vorstehenden Ansprüche **dadurch gekennzeichnet, daß** der Hohlkörper eine Schutzröhre zum Schutz druckempfindlicher Duftstoffe umfaßt.

28. Verwendung der Vorrichtung nach einem der Ansprüche 1 bis 27 zur Begleitung von visuellen und/oder akustischen Ereignissen, insbesondere Femsehfilmen, Videofilmen, Musikdarbietungen, wobei die Vorrichtung den Ereignissen zugeordnete Stoffe in die Luft abgibt.

29. Verwendung der Vorrichtung nach einem der Ansprüche 1 bis 27 zur Bereitstellung von Düften in einer Produktinformationseinheit.

30. Abspielgerät, umfassend:
- eine Vorrichtung (5) zur Bereitstellung von in die Luft oder in Luftgemische abzugebenden Stoffen, insbesondere von Duftstoffen, nach einem der vorstehenden Ansprüche 1 bis 27,
- eine Einrichtung (66) zur Erzeugung eines Gasstromes und
- Mittel, um den Gasstrom in einzelne der Kanäle (3) oder Hohlräume der Vorrichtung (5) zur Bereitstellung von in die Luft oder in Luftgemische abzugebenden Stoffen selektiv einzuleiten.

31. Abspielgerät nach Anspruch 30, **dadurch gekennzeichnet, daß** sie eine Einrichtung aufweist, um die Vorrichtung bewegbar zu lagern.

32. Abspielgerät nach Anspruch 30, **dadurch gekennzeichnet, daß** sie eine Einrichtung aufweist, um die Vorrichtung stationär zu lagern und Mittel, um Gas einer oder mehrerer der Öffnungen eines Hohlkörpers oder der Kanäle selektiv oder gemeinsam zuzuführen.

33. Abspielgerät nach einem der Ansprüche 30 bis 32, **dadurch gekennzeichnet, daß** die Einrichtung zur Erzeugung eines Gasstroms zumindest eine Pumpe ist.

34. Abspielgerät nach einem der Ansprüche 30 bis 33, **dadurch gekennzeichnet, daß** der Gasstrom einen Luftstrom umfaßt.

35. Abspielgerät nach Anspruch 34, **dadurch gekennzeichnet, daß** der Gasstrom einen Luftstrom umfaßt, dem Helium beigemischt ist.

36. Abspielgerät nach Anspruch 35, **dadurch gekennzeichnet, daß** dem Luftstrom 1 bis 20 Volumen-% Helium beigemischt sind.

## Claims

1. A device for supplying substances to be dispensed into air or air mixtures, in particular scents, with a flat disk-shaped or plate-shaped base body (8,9) that has multiple separate channels (3) running through it essentially parallel to its top and/or bottom sides, with the channels serving to accommodate the substances to be dispensed and containing an inlet port and an outlet port (25), so a stream of gas supplied to the inlet port can flow through, with the inlet and outlet ports of at least one channel (3) being sealed in a gastight manner until the substance is dispensed and/or the substance being placed under gastight seal in at least one channel (3) in a reservoir that releases the substance only at the time when it is dispensed.

2. The device according to claim 1, **characterized in that** at least one inlet and outlet port is sealed with a seal that can be destroyed.

3. The device according to claim 1, **characterized in that** the sealed channel contains at least the substance to be dispensed and/or an inert gas in its interior.

4. The device according to one of the preceding claims, **characterized in that** the reservoir contains the substance enclosed in a carrier material so that the substance can be released by destroying the carrier material.

5. The device according to one of the preceding claims, **characterized by** actuating means that cause the opening or destruction of the gastight seals of the inlet and/or outlet port and/or destroy the carrier material for the substances in order to activate the dispensing of the substances to the ambient air or the gas stream supplied to the inlet port.

6. The device according to one of the preceding claims, **characterized by** means for controlled destruction of the gastight seal or the carrier material in the hollow body or channel.

7. The device according to claim 6, **characterized in that** the means for controlled destruction are provided in the device itself.

8. The device according to claim 6, **characterized in that** the means for destruction are provided separately from the device in such a way that they can be brought into operative connection with the device on activation of the dispensing of the substances, with the means preferably being designed in such a way that they become active on insertion of the device into a device for dispensing the gaseous substances.

9. A device, in particular according to one of the preceding claims, for supplying substances, in particular scents, to be dispensed in the form of gas,
**characterized by**
- at least one hollow body (8,9) which is provided with at least one gas exchange port (25) and whose interior (3) can be purged by a gas flow;
- at least one reservoir (19) arranged in the hollow body (8,9) for the substance which is to be dispensed into an environment and which is enclosed by a carrier material (10, 22; 11) so that the substance can be released by destroying at least part of the carrier material (10, 22; 11);
- means (23) for controlled destruction of the carrier material (10, 22; 11) in the hollow body, wherein the means for controlled destruction are provided in the device itself, or the means for destruction are provided separately from the device in such a way that they can be brought into operative connection with the device on activation of the dispensing of the substances, with the means preferably being designed in such a way that they become active on insertion of the device into a device for dispensing the gaseous substances.

10. The device according to claim 9, **characterized in that** the hollow bodies are designed as channels.

11. The device according to one of claims 9 or 10, **characterized in that** the means for controlled destruction of the airtight seals and/or the carrier material are mechanical and/or pneumatic means.

12. The device according to one of claims 4 to 11, **characterized in that** the hollow body or the channels have wall elements with which the carrier material is in operative connection and which can be displaced toward each other.

13. The device according to one of claims 4 to 12, **characterized in that** the carrier material is arranged next to the gas exchange port of the hollow body or the inlet port of the channel in such a way that a stream of gas directed into the interior of the hollow body or channel through the gas exchange port or the gas inlet port destroys at least a part of the carrier material arranged in the hollow body or channel.

14. The device according to one of claims 4 to 13, **characterized in that** the carrier material is arranged in the hollow body or in one of the channels so that it essentially seals off the cross section, so the carrier material is broken open to release the substance when gas is blown into the gas inlet port of the hollow body for the first time.

15. The device according to one of the preceding claims, **characterized in that** an absorption medium impregnated or enriched with the substance to be dispensed is arranged in the hollow body or channel.

16. The device according to one of the preceding claims, **characterized in that** the reservoir contains microcapsules which are at least partially enclosed by the carrier material and in which the substance to be released is enclosed.

17. The device according to one of the preceding claims, **characterized in that** a multitude of hollow bodies or the base body are/is composed of a top cover layer (8), a bottom cover layer (9) and the dividing elements (7) which separate the hollow bodies or channels from each other.

18. The device according to one of claims 4 to 17, **characterized in that** the carrier material contains a sheathing layer (11), another sheathing layer (12) detachably connected to the sheathing layer (11), and an adhesive joining the two sheathing layers (11, 12) together, with the reservoir embedded in them.

19. The device according to one of the preceding claims, **characterized in that** at least one gas exchange port of the hollow body can be sealed.

20. The device according to one of the preceding claims 2 to 9, **characterized in that** a plurality of hollow bodies or channels are arranged side by side.

21. The device according to claim 20, **characterized in that** the hollow bodies or channels are arranged so they run essentially radially with respect to a center.

22. The device according to claim 20, **characterized in that** the hollow bodies or channels are arranged so they run at least partially at an inclination or parallel to each other.

23. The device according to one of the preceding claims, **characterized in that** at least a plurality of hollow bodies or channels are arranged side by side and/or one above the other.

24. The device according to one of the preceding claims, **characterized in that** the reservoir takes up only a portion of the hollow body.

25. The device according to one of the preceding claims, **characterized in that** it is composed at least in part of biodegradable material.

26. The device according to one of the preceding claims, **characterized in that** the reservoir is attached in the hollow body or channel at least next to one of the ports in the hollow body in such a way that it essentially seals off the cross section of the hollow body or channel, as well as having means to supply gas in a controlled manner to at least one port of the hollow body.

27. The device according to one of the preceding claims, **characterized in that** the hollow body comprises a protective tube for protecting pressure-sensitive scents.

28. Use of the device according to one of claims 1 to 27 for accompanying visual and/or acoustic events, in particular television films, video films, music presentations, wherein said device dispenses substances assigned to the events into the air.

29. Use of the device according to one of claims 1 to 27 for providing scents in a product information unit.

30. A player apparatus, comprising:
- a device (5) for supplying substances to be dispensed into air or air mixtures, in particular scents, according to one of the preceding claims 1 to 27,
- equipment (66) for generating a gas stream, and
- means for selectively introducing the stream of gas into individual ones of the channels (3) or cavities of the device (5) for supplying substances to be dispensed into air or air mixtures.

31. The player apparatus according to claim 30, **characterized in that** it has equipment for storing the device in a movable manner.

32. The player apparatus according to claim 30, **characterized in that** it has equipment for storing the device in a stationary manner and means for supplying gas to one or more of the ports of a hollow body or the channels, either selectively or jointly.

33. The player apparatus according to one of claims 30 to 32, **characterized in that** the equipment for generating a stream of gas is at least a pump.

34. The player apparatus according to one of claims 30 to 33, **characterized in that** the gas stream comprises an air stream.

35. The player apparatus according to claim 34, **characterized in that** the gas stream comprises an air stream to which helium is admixed.

36. The player apparatus according to claim 35, **characterized in that** 1 to 20 vol% helium is admixed to the air stream.

## Revendications

1. Dispositif pour acheminer des substances à diffuser dans l'air ou dans des mélanges d'air, en particulier des substances odoriférantes, comprenant un corps de base (8, 9) plat en forme de disque ou de plaque, traversé essentiellement parallèlement à sa face supérieure et/ou sa face inférieure par plusieurs canaux séparés (3) qui servent à recevoir les substances à diffuser et comportent chacun une ouverture d'entrée et une ouverture de sortie (25) et qui peuvent être traversés par un courant de gaz introduit à l'ouverture d'entrée, l'ouverture d'entrée et l'ouverture de sortie d'un canal au moins (3) étant fermées de manière étanche aux gaz jusqu'à la diffusion de la substance, et/ou la substance est introduite de façon étanche aux gaz dans au moins un canal (3) dans un accumulateur qui libère la substance uniquement à l'instant de sa diffusion.

2. Dispositif selon la revendication 1, **caractérisé en ce qu'**au moins une ouverture d'entrée et de sortie est refermée par un sceau destructible.

3. Dispositif selon la revendication 1, **caractérisé en ce que** le canal fermé contient à l'intérieur au moins la substance à diffuser et/ou un gaz inerte.

4. Dispositif selon l'une des revendications précédentes, **caractérisé en ce que** l'accumulateur contient la substance dans un matériau porteur et de manière incluse de telle sorte que la substance est libérable par destruction du matériau porteur.

5. Dispositif selon l'une des revendications précédentes, **caractérisé par** des moyens d'actionnement qui produisent une ouverture ou une destruction des obturations étanches aux gaz dans l'ouverture d'entrée et/ou l'ouverture de sortie, et/ou qui détruisent le matériau porteur pour les substances, afin d'activer la diffusion de la substance vers l'air environnant ou vers un courant de gaz amené à l'ouverture d'entrée.

6. Dispositif selon l'une des revendications précédentes, **caractérisé par** des moyens pour la destruction ciblée de l'obturation étanche aux gaz ou du matériau porteur dans le corps creux ou dans le canal.

7. Dispositif selon la revendication 6, **caractérisé en ce que** les moyens pour la destruction ciblée sont montés dans le dispositif lui-même.

8. Dispositif selon la revendication 6, **caractérisé en ce que** les moyens pour la destruction sont prévus de manière séparée du dispositif, de sorte qu'ils peuvent être amenés en coopération avec le dispositif pour activer la diffusion des substances, lesdits moyens étant de préférence ainsi réalisés qu'ils deviennent actifs lorsqu'on met en place le dispositif dans un appareil pour la diffusion des substances gazeuses.

9. Dispositif, en particulier selon l'une des revendications précédentes, pour acheminer des substances à diffuser gazeuses, en particulier des substances odoriférantes, **caractérisé par** :
-- au moins un corps creux (8, 9) pourvu d'au moins une ouverture d'échange de gaz (25) et dont l'intérieur (3) peut être traversé par un écoulement de gaz ;
-- au moins un accumulateur (19), agencé dans le corps creux (8, 9) pour la substance à diffuser dans un environnement, laquelle est enfermée dans un matériau porteur (10, 22 ; 11) de telle manière que la substance peut être libérée par destruction d'une partie au moins du matériau porteur (10, 22 ; 11) ; et
-- des moyens (23) pour la destruction ciblée du matériau porteur (10, 22 ; 11) dans le corps creux, lesdits moyens pour la destruction ciblée sont montés dans le dispositif lui-même, ou bien les moyens pour la destruction sont prévus de manière séparée du dispositif de telle sorte qu'ils peuvent être amenés en coopération avec le dispositif lors de l'activation de la diffusion des substances, lesdits moyens étant de préférence ainsi réalisés qu'ils deviennent actifs lors de la mise en place du dispositif dans un appareil pour la diffusion des substances gazeuses.

10. Dispositif selon la revendication 9, **caractérisé en ce que** les corps creux sont réalisés sous forme de canaux.

11. Dispositif selon l'une ou l'autre des revendications 9 et 10, **caractérisé en ce que** les moyens pour la destruction ciblée des obturations étanches aux gaz et/ou du matériau porteur sont des moyens mécaniques et/ou pneumatiques.

12. Dispositif selon l'une des revendications 4 à 11, **caractérisé en ce que** le corps creux ou les canaux comportent des éléments de paroi susceptibles d'être déplacés les uns par rapport aux autres, avec lesquels le matériau porteur est relié en termes d'action.

13. Dispositif selon l'une des revendications 4 à 12, **caractérisé en ce que** le matériau porteur est agencé au voisinage de l'ouverture d'échange de gaz du corps creux ou de l'ouverture d'entrée du canal, de telle façon qu'un jet de gaz dirigé à travers l'ouverture d'échange de gaz ou à travers l'ouverture d'entrée de gaz vers l'intérieur du corps creux ou du canal détruit au moins une partie du matériau porteur agencé dans le corps creux ou dans le canal.

14. Dispositif selon l'une des revendications 4 à 13, **caractérisé en ce que** le matériau porteur est agencé dans le corps creux ou dans l'un des canaux de manière à refermer sensiblement sa section transversale, et de sorte que lors d'une première insufflation de gaz dans l'ouverture d'entrée de gaz du corps creux, le matériau porteur est brisé pour libérer la substance.

15. Dispositif selon l'une des revendications précédentes, **caractérisé en ce qu'**un milieu d'absorption enrichi ou imbibé avec la substance à diffuser est agencé dans le corps creux ou dans le canal.

16. Dispositif selon l'une des revendications précédentes, **caractérisé en ce que** l'accumulateur du matériau porteur contient au moins partiellement des micro-capsules incluses dans lesquelles est enfermée la substance à libérer.

17. Dispositif selon l'une des revendications précédentes, **caractérisé en ce qu'**une pluralité de corps creux ou le corps de base sont constitués d'une couche de couverture (8) du côté supérieur, d'une couche de couverture (9) du côté inférieur, ainsi que de barrettes de séparation (7) qui délimitent les corps creux ou les canaux les uns vis-à-vis des autres.

18. Dispositif selon l'une des revendications 4 à 17, **caractérisé en ce que** le matériau porteur contient une couche enveloppe (11), une seconde couche enveloppe (12) reliée de manière détachable à la première couche enveloppe (11), ainsi qu'un produit d'adhésion qui relie les deux couches enveloppe (11, 12) et dans lequel est noyé l'accumulateur.

19. Dispositif selon l'une des revendications précédentes, **caractérisé en ce qu'**une ouverture d'échange de gaz du corps creux au moins est susceptible d'être scellée.

20. Dispositif selon l'une des revendications 2 à 9, **caractérisé en ce qu'**une pluralité de corps creux ou de canaux sont agencés les uns à côté des autres.

21. Dispositif selon la revendication 20, **caractérisé en ce que** les corps creux ou les canaux sont agencés de manière à s'étendre essentiellement radialement, par référence à un centre.

22. Dispositif selon la revendication 20, **caractérisé en ce que** les corps creux ou les canaux sont agencés de façon à s'étendre au moins partiellement en oblique ou parallèlement les uns aux autres.

23. Dispositif selon l'une des revendications précédentes, **caractérisé en ce que** la majorité au moins des corps creux ou des canaux sont agencés les uns à côté des autres et/ou les uns au-dessus des autres.

24. Dispositif selon l'une des revendications précédentes, **caractérisé en ce que** l'accumulateur occupe seulement une partie du corps creux.

25. Dispositif selon l'une des revendications précédentes, **caractérisé en ce que** celui-ci est réalisé au moins partiellement en matériau biodégradable.

26. Dispositif selon l'une des revendications précédentes, **caractérisé en ce que** l'accumulateur est fixé dans le corps creux ou dans le canal est au moins au voisinage de l'une des ouvertures du corps creux de telle façon qu'il referme essentiellement la section transversale du corps creux ou du canal, et comprend des moyens pour admettre du gaz de façon commandée vers au moins une ouverture du corps creux.

27. Dispositif selon l'une des revendications précédentes, **caractérisé en ce que** le corps creux inclut un tube protecteur pour la protection de substances odoriférantes sensibles à la pression.

28. Utilisation du dispositif selon l'une des revendications 1 à 27 pour l'accompagnement d'événements visuels et/ou acoustiques, en particulier de film de télévision, de films vidéo, d'événements musicaux, dans laquelle le dispositif diffuse dans l'air des substances associées aux événements.

29. Utilisation du dispositif selon l'une des revendications 1 à 27 pour l'acheminement d'odeurs dans une unité d'information relative à un produit.

30. Appareil diffuseur, comprenant :
-- un dispositif (5) pour acheminer des substances à diffuser dans de l'air ou dans des mélanges d'air, en particulier des substances odoriférantes, selon l'une des revendications 1 à 27,
-- des moyens (66) pour produire un courant de gaz, et
-- des moyens pour introduire le courant de gaz de manière sélective dans des canaux individuels (3) ou des cavités individuelles du dispositif (5) pour acheminer des substances à diffuser dans de l'air ou dans des mélanges d'air.

31. Appareil selon la revendication 30, **caractérisé en ce qu'**il comprend des moyens pour monter le dispositif de façon mobile.

32. Appareil selon la revendication 30, **caractérisé en ce qu'**il comprend des moyens pour monter le dispositif de manière stationnaire, et des moyens pour admettre un gaz à l'une ou à plusieurs des ouvertures d'un corps creux ou des canaux, de manière sélective ou conjointe.

33. Appareil selon l'une des revendications 30 à 32, **caractérisé en ce que** les moyens pour produire un courant de gaz sont constitués par au moins une pompe.

34. Appareil selon l'une des revendications 30 à 33, **caractérisé en ce que** le courant de gaz comprend un courant d'air.

35. Appareil selon la revendication 34, **caractérisé en ce que** le courant de gaz comprend un courant d'air auquel est mélangé de l'hélium.

36. Appareil selon la revendication 35, **caractérisé en ce que** l'on mélange de 1 à 20 volumes % d'hélium dans le courant d'air.
